# EUROPEAN PATENT SPECIFICATION

(11) **EP 0 644 768 B1**
(45) Date of publication and mention of the grant of the patent: **21.11.2001**
(21) Application number: 93916500.7
(22) Date of filing: 11.06.1993
(51) Int. Cl.: A61K 38/00, A61K 39/395, C07K 7/00, C07K 2/00, G01N 33/53

(54) **METHODS AND COMPOSITIONS FOR INHIBITING ENDOTHELIAL CELL AND FIBRINOGEN MEDIATED INFLAMMATION**
VERFAHREN UND ZUSAMMENSETZUNGEN ZUR VERHINDERUNG VON ENTZÜNDUNGEN, DIE DURCH ENDOTHELZELLEN UND FIBRINOGEN AUSGELÖST WERDEN
PROCEDES ET COMPOSITIONS D'INHIBITION D'INFLAMMATIONS PROVOQUEES PAR DES CELLULES ENDOTHELIALES ET PAR LE FIBRINOGENE

(30) Priority: 11.06.1992 US 898117
(43) Date of publication of application: 29.03.1995
(73) Proprietor: THE SCRIPPS RESEARCH INSTITUTE, La Jolla, CA 92037 (US)
(72) Inventor: LANGUINO, Lucia, R., La Jolla, CA 92037 (US); ALTIERI, Dario, C., La Jolla, CA 92037 (US); PLOW, Edward, F., Solon, OH 44139 (US); GELTOSKY, John, E., Carlsbad, CA 92009 (US)
(74) Representative: Fisher, Adrian John
(86) International application number: US9305610
(87) International publication number: WO9325218

(56) References cited:
- WO-A-90/03400
- WO-A-92/01464
- US-A- 4 650 678
- Immunol. Reviews, Volume 114, issued April 1990, M.A. ARNAOUT, "Leukocyte Adhesion Molecules Deficiency: Its Structural Basis, Pathophysiology and Implications for Modulating the Inflammatory Response", pages 145-180, see entire document.
- J. Clin. Invest., Volume 75, issued January 1985, E. DEJANA et al., "Interaction Between Fibrinogen and Cultured Endothelial Cells", pages 11-18, see entire document.
- PNAS, Volume 84, issued September 1987, D.A. CHERESH, "Human Endothelial Cells Synthesize and Express an Arg-Gly-Asp-Directed Adhesion Receptor Involved in Attachment to Fibrinogen and Von Willebrand Factor", pages 6471-6475, see entire document.
- Cell, Volume 58, issued 08 September 1989, D.A. CHERESH et al., "Recognition of Distinct Adhesive Sites on Fibrinogen by Related Integrins on Platelets and Endothelial Cells", pages 945-953, see entire document.
- J. Clin. Invest., Volume 90, issued December 1992, M. GE et al., "Fibrinogen Degradation Product Fragment D Induces Endothelial Cell Detachment by Activation of Cell-Mediated Fibrinolysis", pages 2508-2516, see entire document.
- J. Biol. Chem., Volume 256, Number 15, issued 10 August 1981, D.S. FAIR et al., "Immunochemical Mapping of the Conformation of Human Fibrinogen", pages 8018-8023, see entire document.
- TIBTECH, Volume 11, issued February 1993, W.J. HARRIS et al., "Therapeutic Antibodies - the Coming of Age", pages 42-44, see entire document.

## Description

### Technical Field

The present invention contemplates the use of compositions to inhibit fibrinogen binding to endothelial cells for the purpose of inhibiting endothelial cell and fibrinogen mediated inflammation.

### Background

Adhesion of leukocytes to vascular endothelium is one of the earliest events in a variety of immune-inflammatory reactions. The process participates in vascular occlusions and contributes to atherothrombotic lesions. At the molecular level, leukocyte adhesion to endothelial cells is a redundant mechanism, supported by the regulated recognition of a disparate set of membrane receptors, including integrins, expressed on both leukocytes and resting or cytokine-activated endothelial cells.

Integrins are a functionally and structurally related group of receptors that interact with a wide variety of ligands including extracellular matrix glycoproteins, complement and other cells. Integrins participate in cell-matrix and cell-cell adhesion in many physiologically important processes including embryological development, hemostasis, thrombosis, wound healing immune and nonimmune defense mechanisms and oncogenic transformation. See Hynes, Cell, 48:549-554 (1987). The majority of integrins participating in dynamic cell adhesion, bind a tripeptide, arginine-glycine-aspartic acid (RGD), present in their ligand, causing cell adhesion. SeeRuoslahti et al., Science, 238:491-497 (1987).

Mac-1 (CD11b/CD18) is an integrin receptor found predominantly on macrophages and granulocytes. Like all integrin receptors, Mac-1 is a heterodimeric, transmembrane glycoprotein composed of non-covalently associated alpha and beta subunits.

Mac-1 mediates neutrophil/monocyte adhesion to vascular endothelium and phagocytosis of complement-opsonized particles. Antibodies to the Mac-1 receptor alter neutrophil function in vivo including inhibiting neutrophil migration into inflammatory sites. See Price et al., J. Immunol., 139:4174-4177 (1987). Mac-1 also functions as a receptor for fibrinogen in a reaction linked to fibrin deposition on the monocyte surface. See Altieri et al., J. Cell Biol., 107:1893-1900 (1988); Wright et al., Proc. Natl. Acad. Sci. USA, 85:7734-7738 (1988); Trezzini et al., Biochem. Biophys. Res. Commun., 156:477-484 (1988) and Gustafson et al., J. Cell Biol., 109:377-387 (1989).

Fibrinogen is a complex molecule of approximately 340,000 daltons and consists of three pairs of subunit polypeptides, called the α, β and gamma chains. These individual chains are held together by several disulfide bonds. The proteolytic digestion of fibrinogen by plasmin produces fragments A, B, C, D and E, all having a molecular weight of less the 85,000 daltons. See Pizzo et al., J. Biol. Chem., 247:636-645 (1972).

Further proteolytic digestion of fibrinogen by plasmin produces a D₃₀ fragment with a molecular weight of about 30,000 daltons containing portions of the α, β and gamma chains of fibrinogen. See Furlan et al., Biochim. Biophys. Acta., 400:95-111 (1975).

The deposition of fibrinogen on the leukocyte surface occurs in a variety of inflammatory responses such as delayed type hypersensitivity, incompatible transplant rejection and the physiopathology of vascular obstruction and atherogenesis. See Geczy et al., J. Immunol., 130:2743-2749 (1983); Hooper et al., J. Immunol., 126:1052-1058 (1981); Colvin et al., J. Immunol., 114:377-387 (1975); Hattler et al., Cell Immunology, 9:289-295 (1973); Gerrity, R.G., Am. J. Pathol., 103:181-190 (1981) and Am. J. Pathol., 103:191-200 (1981); and Shelley et al., Nature, 270:343-344 (1977).

Interactions of fibrinogen on cell surface receptors of endothelial cells have been described. Languino et al., Blood, 73:734 (1989) describe the binding of fibrinogen to endothelial cells by an RGD-dependent mechanism. It is generally believed that the vitronectin receptor is the major endothelial receptor for fibrinogen. Cheresh et al, Proc. Natl. Acad. Sci. USA, 84:6471-6475 (1987). Other endothelial cell receptors reported to bind fibrinogen include cell surface bound transglutaminase, and an 130 kilodalton receptor that binds to fibrin peptides. Erban et al., J. Biol. Chem., 267:2451 (1992).

Also on the surface of endothelial cells is an intercellular adhesion molecule 1 (ICAM-1) that has been described by Springer, Nature, 346:425-433 (1990), and has been shown to bind the leukocyte integrin LFA-1.

Recently, the interaction of fibrinogen with the Mac-1 receptor of leukocytes has been shown to be a dynamic cell adhesion reaction involving the recognition of the tripeptide RGD within fibrinogen by the Mac-1 receptor similar to the interaction of fibrinogen with the integrin receptors on platelets and endothelial cells. See Altieri et al., J. Clinic Invest., 78:968-976 (1986); Pytela et al., Science, 231:1559-1562 (1986) and Ruoslahti et al., Science, 238:491-497 (1987) and Cell, 44:517-518 (1986). International PCT Application No. PCT/US91/05096 discloses compositions comprising D₃₀ and variants thereof.

### Brief Description of the Invention

It has now been discovered that fibrinogen binds to both the Mac-1 receptor on leukocytes and to an endothelial cell receptor (ECR), thereby bridging between the leukocyte and the endothelial cell during the process of inflammation. Inflammation arising from this bridging event is referred to as endothelial cell/fibrinogen-mediated inflammation. The ECR is ICAM-1, which is an RGD-independent, fibrinogen specific receptor.

In this specification, the term "ECR" refers to ICAM-1.

The invention describes novel compositions defining the binding sites for the interaction between ECR and fibrinogen.

Thus, a composition is contemplated comprising a therapeutically effective amount of a substantially pure and pharmaceutically acceptable fibrinogen homolog capable of binding to ECR and inhibiting Fg binding to endothelial cells. In the compositions which are claimed herein, homolog is neither D₃₀ nor a variant thereof.

Also contemplated is a composition comprising a therapeutically effective amount of a substantially pure and pharmaceutically acceptable ICAM-1 homolog capable of binding to fibrinogen and inhibiting fibrinogen binding to endothelial cells.

The invention also describes a monoclonal antibody that immunoreacts with ICAM-1, but does not immunoreact with the vitronectin receptor, such that the monoclonal antibody preferentially inhibits fibrinogen binding to stimulated endothelial cells.

A monoclonal antibody is also described that immunoreacts with fibrinogen and that preferentially inhibits fibrinogen binding to stimulated endothelial cells.

Also described is a method of inhibiting fibrinogen (Fg) binding to endothelial cells comprising contacting the endothelial cells with a Fg-binding inhibiting amount of a pharmaceutically acceptable composition comprising a substantially pure homolog selected from the group consisting of a Fg homolog and an ICAM-1 homolog.

The method is useful for inhibiting fibrinogen/ endothelial cell-mediated inflammation in a patient and comprises administering to a patient a therapeutically effective amount of a pharmaceutically acceptable composition comprising a substantially pure homolog selected from the group consisting of a Fg homolog and an ICAM-1 homolog.

The invention also describes a method of detecting the amount of a fibrinogen (Fg) homolog in a liquid sample comprising:
(a) admixing a sample of stimulated endothelial cells with a predetermined amount of a liquid sample containing a Fg homolog and a predetermined amount of labelled Fg homolog to form a competition reaction admixture;
(b) maintaining the reaction admixture for a predetermined time period sufficient for any Fg homolog present in said composition to bind to the endothelial cells and form an endothelial cell:Fg homolog complex and to allow the labelled Fg homolog to bind to the endothelial cells to form a labelled endothelial cell:Fg homolog complex; and
(c) assaying for the amount of labelled endothelial cell:Fg homolog complex formed in step (b) thereby detecting the amount of a Fg homolog in the composition.

Also described is a method of screening for compositions effective at inhibiting fibrinogen binding to ICAM-1 comprising the steps of:
a) admixing in an inhibition reaction admixture preselected amounts of a putative inhibitor composition, a fibrinogen homolog, and an ICAM-1 homolog;
b) maintaining the admixture under conditions sufficient for the ICAM-1 homolog to bind to the Fg homolog and form an ICAM-1 homolog:Fg homolog complex; and
c) measuring the amount of ICAM-1 homolog:Fg homolog complex formed in step (b), and thereby the effectiveness of the inhibitor composition.

Also described is a method for preparing substantially pure ICAM-1 comprising the steps of:
(a) providing an aqueous detergent composition containing at least ICAM-1;
(b) contacting the ICAM-1-containing composition with a fibrinogen-immobilized matrix comprising fibrinogen affixed to a solid support, wherein the contacting is conducted under conditions sufficient for the ICAM-1 to bind to the fibrinogen and form a solid phase ICAM-1:fibrinogen complex;
(c) washing the solid support and the complex with an aqueous wash buffer comprising Mg⁺⁺, Mn⁺⁺ and an RGD-containing polypeptide under conditions sufficient to elute any proteins bound to fibrinogen in an RGD-dependent manner, wherein the wash buffer is substantially free from Ca⁺⁺; and
(d) eluting the ICAM-1 from the solid support using an aqueous buffer comprising Mg⁺⁺, Mn⁺⁺ and EDTA, to form the substantially pure ICAM-1.

### Brief Description of the Drawings

In the drawings, forming a portion of this disclosure:

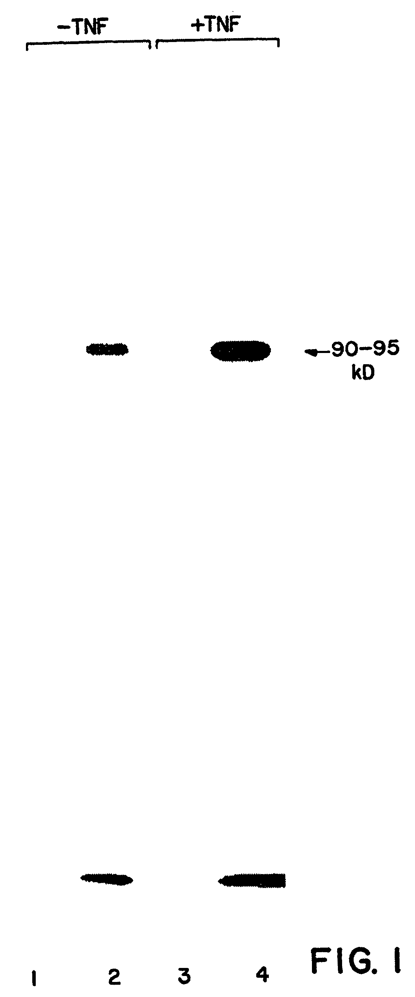

Figure 1 illustrates the autoradiographic results of electrophoresis of aliquots of peak fractions from both the RGD and EDTA elutions of cell lysate supernatants prepared from cells either left untreated or treated with TNF as described in Example 2A. Lanes 1 and 3 show the RGD-eluted receptors isolated from cell lysates respectively prepared from untreated or TNF-treated cells. The characteristic pattern of VNR is present in lane 3. Lanes 2 and 4 respectively show the EDTA-eluted ECR isolated from untreated and TNF-treated cells having a molecular weight band of approximately 90-95 kD, the intensity of which is enhanced about 3-5 fold as a result of the induction of ECR expression by exposure to TNF.

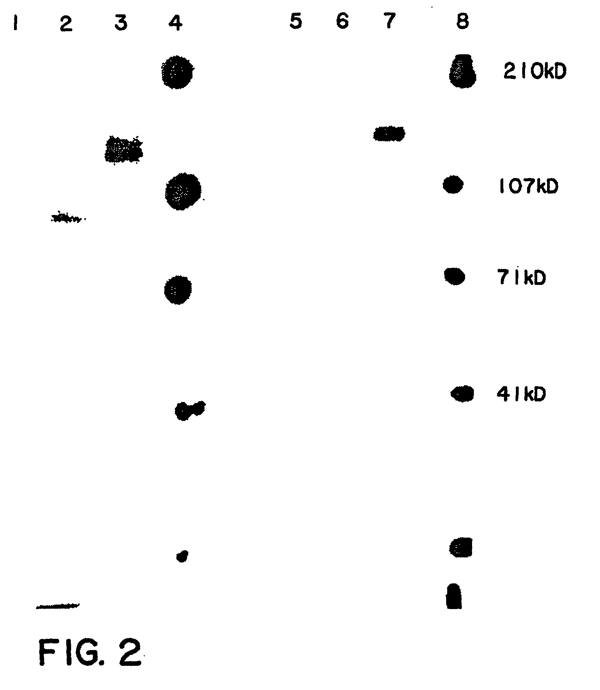

Figure 2 illustrates the results of autoradiographic exposure of the electrophoresed ¹²⁵I-labelled receptors isolated from sequential affinity chromatography of ¹²⁵I-labelled HUVEC cell lysate supernatants over an RGD Sepharose™ followed by a fibrinogen Sepharose™ column. The lanes are labelled 1-8. Lanes 1 through 4 show migration of proteins under reducing conditions while lanes 5 through 8 show the migration of identical aliquots run under nonreducing conditions. ¹²⁵I-labelled molecular weight standards of 210, 107, 71 and 41 kD, respectively, myosin, beta-galactosidase, bovine serum albumin and ovalbumin are run in lanes 4 and 8.

In lanes 3 and 7, the vitronectin receptor eluted with EDTA from the RGD-Sepharose™ column exhibits the characteristic profile of alpha v/beta 3 under reducing and nonreducing conditions as described in Example 2C. Another integrin beta subunit, beta 1, also shown in lanes 3 and 7, was eluted from the RGD Sepharose™ column with EDTA.

Lanes 1 and 5 show the RGD elution of the flow-through from the first RGD column applied onto the second fibrinogen column. Lane 6 shows the results of EDTA elution following the RGD elution where a single band of approximately 90-95 kD under nonreducing conditions was recovered. Under reducing conditions, the molecular weight of the EDTA-eluted fibrinogen receptor only slightly increased as shown in lane 2.

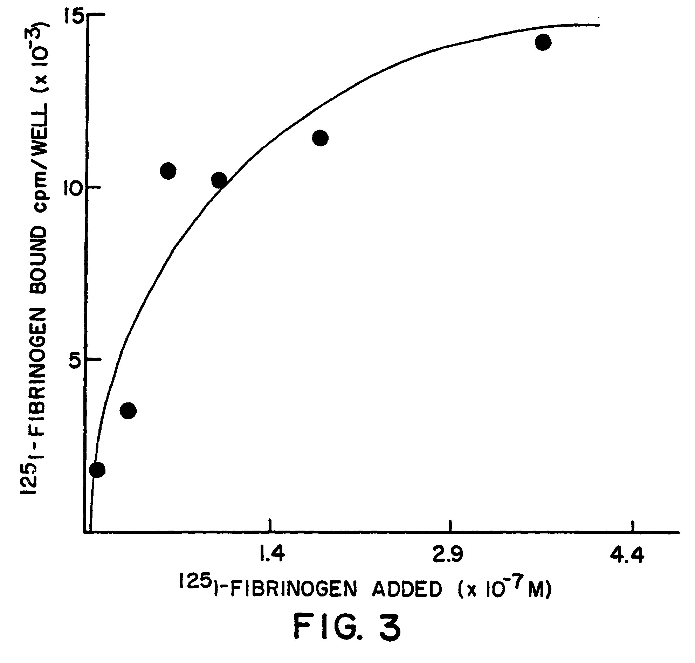

Figure 3 illustrates the dose-response curve of ¹²⁵I-labelled fibrinogen binding to monolayers of HUVEC as described in Example 3A2). The ¹²⁵I-labelled fibrinogen bound in counts per minute (cpm) per well (X 10⁻³) is plotted on the Y-axis against increasing concentrations of ¹²⁵I-labelled fibrinogen (X 10⁻⁷ M) on the X-axis. The data shows that ¹²⁵I-labelled fibrinogen binds saturably at a concentration of approximately 0.36 *µ*M to monolayers of unstimulated HUVEC.

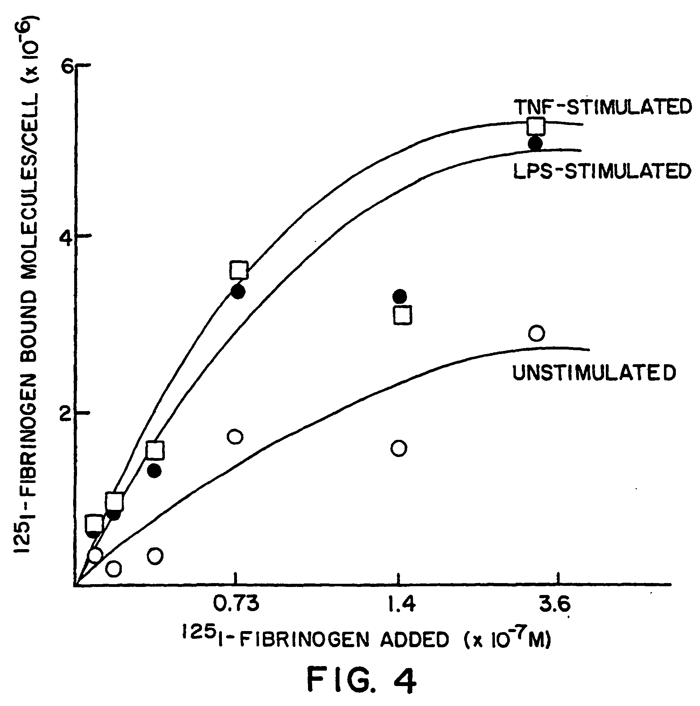

Figure 4 illustrates the dose-response curve of ¹²⁵I-labelled fibrinogen binding to unstimulated and either TNF- or LPS-stimulated HUVEC as described in Example 3A3). ¹²⁵I-labelled fibrinogen bound in molecules per cell (X 10⁻⁶) is plotted on the Y-axis against increasing concentrations of ¹²⁵I-labelled fibrinogen (X 10⁻⁷ M) on the X-axis. Under stimulation with either TNF or LPS, the number of labelled fibrinogen molecules bound per cell doubled in comparison to those bound to unstimulated cells.

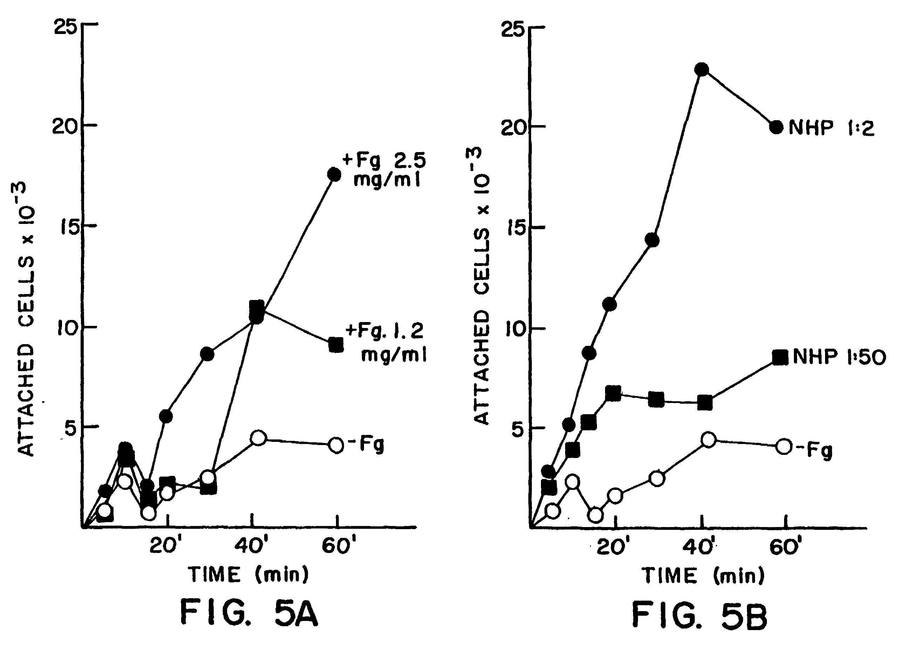

Figure 5 illustrates the dose and time dependent effects on the ability of fibrinogen to mediate the binding of ⁵¹Cr-labelled THP-1 cells to HUVEC. The results of these experiments are shown in Figure 5A and Figure 5B where the data is expressed as numbers of ⁵¹Cr-labelled THP-1 cells (X 10⁻³) on the Y-axis plotted against the assay time on the X-axis. Figure 5A shows the effect of different concentrations of purified fibrinogen admixed with THP-1 cells compared to the absence of fibrinogen (labelled as Fg) in mediating the binding to HUVEC cultures over a 60 minute period as described in Example 3B1). Figure 5B shows results of similar assays done in the presence of dilutions of normal human plasma (NHP).

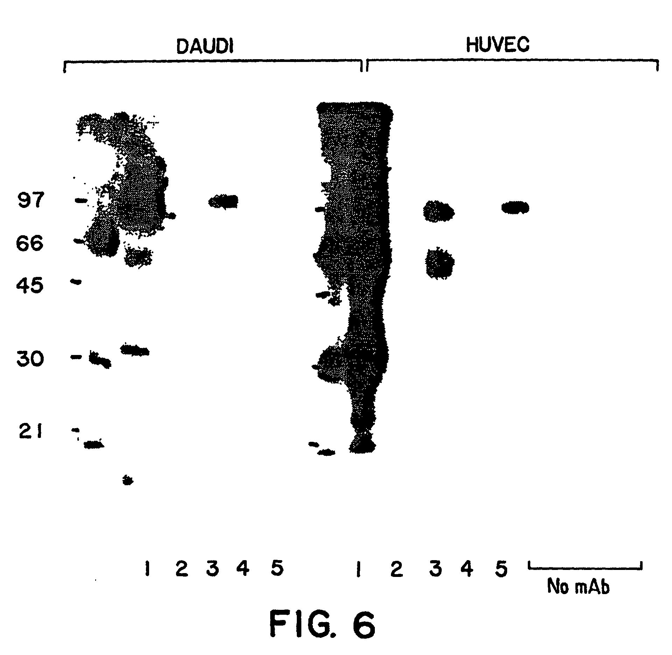

Figure 6 illustrates the results of the Westerr. blot as described in Example 4E. Radiolabelled molecular weight markers of 97, 66, 45, 30 and 21 kD shown in lane left of the first set of 5 Daudi lanes and left of the second set of 8 HUVEC lanes. Lanes designated 1-5 at the bottom of the blot for both Daudi and HUVEC were respectively immunoreacted with 2E1, PMI-I, affinity purified 14E11, 14E11 culture supernatants and the anti-ICAM-1 BD monoclonal antibodies. The three extra lanes in the HUVEC side of the blot show the nonspecific background when no primary antibody is used.

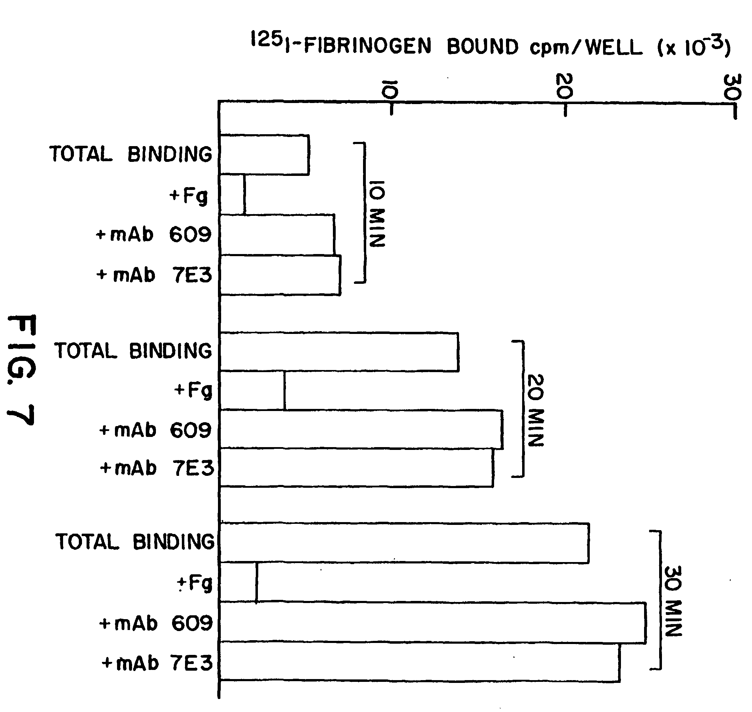

Figure 7 illustrates in bar graphs the inhibition of binding of ¹²⁵I-labelled fibrinogen to HUVEC cultures in the presence of 50 fold excess of unlabelled fibrinogen (Fg) over time as described in Example 5A1). The amount of radioactivity associated with the cells after harvesting is expressed on the Y-axis as cpm/well (X 10⁻³). The noninhibitory effects of exposure to the monoclonal antibodies directed against VNR, designated mAb 609 and mAb 7E3, is also shown. Total binding of ¹²⁵I-labelled fibrinogen in the absence of admixed inhibitors is also shown.

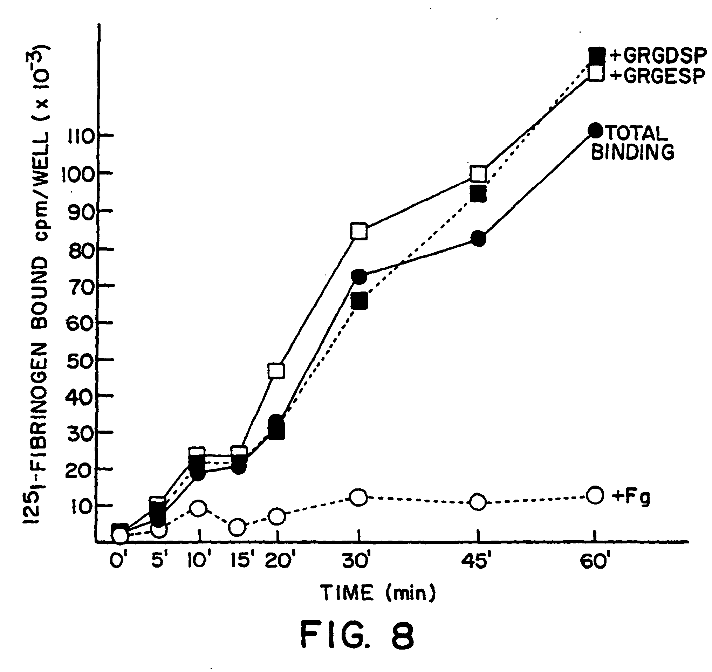

Figure 8 illustrates the effects of exposure to RGD- and RGE-containing peptides on the binding of 125I-labelled fibrinogen to HUVEC as described in Example 5A1). The amount of ¹²⁵I-labelled fibrinogen bound to HUVEC in cpm/well (X 10⁻³) is plotted on the Y-axis against the length of time labelled fibrinogen was maintained with HUVEC.

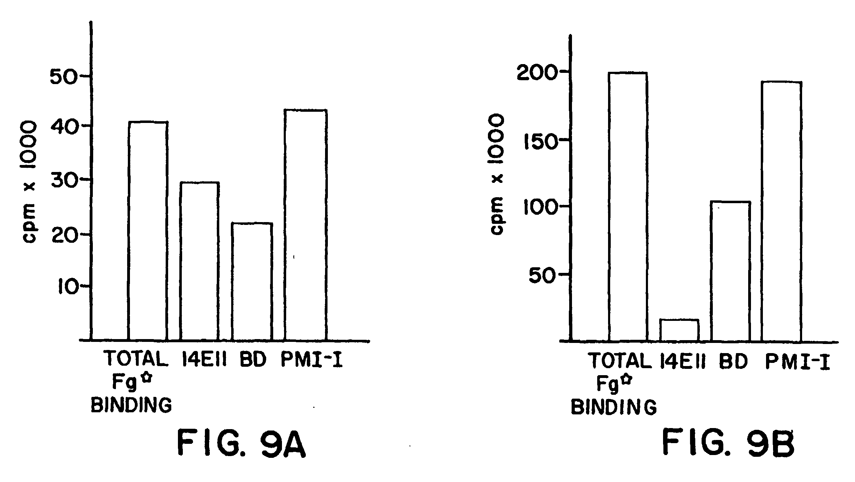

Figure 9 illustrates the inhibition of ¹²⁵I-labelled fibrinogen to unstimulated or TNF-stimulated HUVEC cultures, respectively, Figure 9A and 9B, by treatment of the HUVEC with the monoclonal antibodies, 14E11, BD (anti-ICAM-1, Becton Dickinson) and a control antibody, PMI-I. The experimental protocol is described in Example 5. The data is expressed in a bar graph as the specific binding of ¹²⁵I-labelled fibrinogen in cpm/well (X 10-3) on the Y-axis against the specific treatments on the X-axis.

### Detailed Description of the Invention

### A. General Description

The present invention describes the identification of a novel and specific role for fibrinogen (Fg) in mediating inflammatory processes at endothelial tissues. The role is shown to be a bridging event between the Mac-1 receptor on leukocytes and other Mac-1 receptor-bearing cells and a class of molecules on endothelial cells referred to herein as an endothelial cell receptor (ECR). The interaction of Fg with the ECR is shown to be a unique RGD-independent binding interaction, different from the known binding of Fg to the vitronectin receptor, and to other endothelial cell surface receptors such as transglutaminase or the 130 kilodalton receptor which binds to fibrin-derived peptides.

Insofar as the Fg bridging event described herein adheres Mac-1-bearing cells to endothelial cells, the mechanism discovered and described herein is distinct from the ICAM-1:Mac-1-dependent pathway of leukocyte adhesion, because of the role played by fibrinogen in the present bridging interaction. The Fg-dependent inflammation pathway described herein is referred to as endothelial cell/fibrinogen-mediated inflammation to emphasize the requirement for fibrinogen in the process.

### B. Homologs

A homolog, as used herein is a macromolecule, typically a protein or polypeptide, that mimics the structure and function of a domain of a protein after which it is modeled. Where a native protein carries multiple structural domains and thereby mediates multiple distinct functions, as with fibrinogen, a homolog mimics a particular domain and is able to interact and compete with the native protein for participation with the mediators of that protein function in which the domain participates.

### 1. Fibrinogen Homologs

Thus, according to the present invention, a fibrinogen homolog, or Fg homolog, is a macromolecule that mimics a region of fibrinogen that binds to endothelial cell receptor (ECR, ICAM-1) in an RGD-independent manner according to this invention. The site on ECR to which fibrinogen binds in an RGD-independent manner is referred to as the "ECR RGD-independent Fg-binding site" or "endothelial cell RGD-independent Fg-binding site", that is, a site on endothelial cells, and on the ECR identified herein, that binds to fibrinogen in an RGD-independent manner. The binding site is characterized as RGD-independent because endothelial cells contain other receptors for binding fibrinogen which mediate binding through the RGD-containing region of fibrinogen.

A Fg homolog is any macromolecule which is capable of binding to the ECR RGD-independent Fg-binding site, and thereby can inhibit Fg binding to the ECR RGD-independent Fg-binding site on endothelial cells, and thereby inhibit RGD-independent Fg binding to endothelial cells. Assays for measuring the binding of a Fg homolog to the ECR RGD-independent Fg-binding site are described in Example 3a. Assays for measuring the inhibition of Fg binding to the ECR RGD-independent Fg-binding site are described in Example 5.

A preferred Fg homolog is a fragment of fibrinogen that contains the region of Fg that binds to the ECR RGD-independent Fg-binding site. More preferably, the Fg homolog does not bind to the Mac-1 receptor. Such Fg fragments can be proteolytic fragments of Fg, fibrinogen-derived polypeptides, portions of fibrinogen, D₃₀, portions of D₃₀, polypeptides or proteins homologous to D₃₀ or fibrinogen containing non-natural amino acid derivatives or non-proteinaceous side chains, analogs or chemical derivatives of either D₃₀, fibrinogen, fragments or polypeptides thereof, and conjugates containing a Fg homolog. A preferred Fg homolog is fibrinogen, a proteolytic fragment of fibrinogen, and particularly the D₃₀ fragment of Fg, also referred to as D₃₀.

Although D₃₀ and homolog thereof may be used in the methods of the invention, they are not used in the compositions of claim 1.

The D₃₀ fragment of fibrinogen is produced by proteolytic digestion of fibrinogen. The preparation of D₃₀ has been described by Fair et al., J. Biol. Chem., 256:8018-8023 (1981), Furlaw et al., Biochem. Biophys. Acta., 400:95-11) (1975) and in Example 1. D₃₀ contains partially degraded β and gamma chains and extensively degraded a chains combined by inter-chain disulfide bonds as described by Pizzo et al., J. Biol. Chem., 247:636-645 (1972).

The N-terminus of the Aa chain remnant of D₃₀ originates with amino acid residues Leu¹³⁶, Gln¹³⁷, Lys¹³⁸ and Asn¹³⁹ using the amino acid sequence of the alpha chain described by Doolittle et al., Nature, 280:464-469 (1979).

The Aa chain remnant of D₃₀ does not contain amino acid residues Arg⁹⁵, Gly⁹⁶ and Asp⁹⁷ (RGD) or the amino acid residues Arg⁵⁷², Gly⁵⁷³ and Asp⁵⁷⁴ (RGD) and has a Mw of about 11 to 13 kilodaltons (kDa). The N-termini of the β chain of D₃₀ contains amino acid residues Asp¹³⁴, Asn¹³⁵, Glu¹³⁶, and Asn¹³⁷. The N-terminus of the gamma of D₃₀ contains amino acid residues Met⁸⁹, Leu⁹⁰, Glu⁹¹, and Glu⁹².

A subject Fg homolog includes any analog, fragment or chemical derivative of an active fibrinogen polypeptide as defined herein, so long as the polypeptide is capable of inhibiting Fg binding to the ECR RGD-independent Fg-binding site. Therefore, a polypeptide Fg homolog can be subject to various changes, substitutions, insertions, and deletions where such changes provide for certain advantages in its use. In this regard, a Fg homolog of this invention can contain one or more changes in the polypeptide so long as the homolog retains its function in one or more of the binding and inhibition assays as defined herein.

The term "analog" includes any polypeptide having an amino acid residue sequence substantially identical to a sequence of a Fg homolog or domain of fibrinogen in which one or more residues have been conservatively substituted with a functionally similar residue and which displays the abilities as described herein. Examples of conservative substitutions include the substitution of one non-polar (hydrophobic) residue such as isoleucine, valine, leucine or methionine for another, the substitution of one polar (hydrophilic) residue for another such as between arginine and lysine, between glutamine and asparagine, between glycine and serine, the substitution of one basic residue such as lysine, arginine or histidine for another, or the substitution of one acidic residue, such as aspartic acid or glutamic acid for another.

The phrase "conservative substitution" also includes the use of a chemically derivatized residue in place of a non-derivatized residue provided that such polypeptide displays the requisite binding activity.

"Chemical derivative" refers to a polypeptide having one or more residues chemically derivatized by reaction of a functional side group. Such derivatized molecules include for example, those molecules in which free amino groups have been derivatized to form amine hydrochlorides, p-toluene sulfonyl groups, carbobenzoxy groups, t-butyloxycarbonyl groups, chloroacetyl groups or formyl groups. Free carboxyl groups may be derivatized to form salts, methyl and ethyl esters or other types of esters or hydrazides. Free hydroxyl groups may be derivatized to form 0-acyl or 0-alkyl derivatives. The imidazole nitrogen of histidine may be derivatized to form N-im-benzylhistidine. Also included as chemical derivatives are those peptides which contain one or more naturally occurring amino acid derivatives of the twenty standard amino acids. For example: 4-hydroxyproline may be substituted for proline; 5-hydroxylysine may be substituted for lysine; 3-methylhistidine may be substituted for histidine; homoserine may be substituted or serine; and ornithine may be substituted for lysine. Polypeptides of the present invention also include any polypeptide having one or more additions and/or deletions or residues relative to the sequence of a polypeptide whose sequence is shown herein, so long as the requisite binding activity is maintained.

The term "fragment" refers to any subject polypeptide having an amino acid residue sequence shorter than that the native protein.

When a polypeptide defining a portion of a Fg homolog of the present invention has a sequence that is not identical to the sequence of a portion of fibrinogen, it is typically because one or more conservative or non-conservative substitutions have been made, usually no more than about 30 number percent, more usually no more than 20 number percent, and preferably no more than 10 number percent of the amino acid residues are substituted. Additional residues may also be added at either terminus for the purpose of providing a "linker" by which the polypeptides of this invention can be conveniently affixed to a label or solid matrix, or carrier. Preferably the linker residues do not form Fg homolog epitopes, i.e., are not similar in structure to a Fg homolog.

Labels, solid matrices and carriers that can be used with the Fg homologs of this invention are described hereinbelow.

Amino acid residue linkers are usually at least one residue and can be 40 or more residues, more often 1 to 10 residues, but do not form Fg epitopes. Typical amino acid residues used for linking are tyrosine, cysteine, lysine, glutamic and aspartic acid, or the like. In addition, a subject polypeptide can differ, unless otherwise specified, from the natural sequence of the gamma chain of fibrinogen by the sequence being modified by terminal-NH₂ acylation, e.g., acetylation, or thioglycolic acid amidation, by terminal-carboxylamidation, e.g., with ammonia, methylamine, and the like.

A polypeptide forming a Fg homolog of the present invention can be prepared using the solid-phase synthetic technique initially described by Merrifield, J. Am. Chem. Soc., 85:2149-2154 (1963). Other polypeptide synthesis techniques may be found, for example, in M. Bodanszky et al., Peptide Synthesis, John Wiley & Sons, 2d Ed., (1976) as well as in other reference works known to those skilled in the art. A summary of polypeptide synthesis techniques may be found in J. Stuart and J.D. Young, Solid Phase Peptide Synthesis, Pierce Chemical Company, Rockford, IL, 3d Ed., Neurath, H. et al., Eds., p. 104-237, Academic Press, New York, NY (1976). Appropriate protective groups for use in such syntheses will be found in the above texts as well as in J.F.W. Mcomie, Protective Groups in Organic Chemistry, Plenum Press, New York, NY (1973).

In general, those synthetic methods comprise the sequential addition of one or more amino acid residues or suitably protected amino acid residues to a growing polypeptide chain. Normally, either the amino or carboxyl group of the first amino acid residue is protected by a suitable, selectively removable protecting group is utilized for amino acids containing a reactive side group such as lysine.

Using a solid phase synthesis as an example, the protected or derivatized amino acid is attached to an inert solid support through its unprotected carboxyl or amino group. The protecting group of the amino or carboxyl group is then selectively removed and the next amino acid in the sequence having the complementary (amino or carboxyl) group suitably protected is admixed and reacted under conditions suitable for forming the amid linkage with the residue already attached to the solid support. The protecting group of the amino or carboxyl group is then removed from this newly added amino acid residue, and the next amino acid (suitably protected) is then added, and so forth. After all the desired amino acids have been linked in the proper sequence any remaining terminal and side group protecting groups (and solid support) are removed sequentially or concurrently, to provide the final polypeptide.

Methods and procedures for determining inhibition are well known in the art and include the use of competition assays similar to the antigen competition assays described in Antibodies: A Laboratory Manual, Harlow and Lane, eds., Cold Spring Harbor, NY (1988). For example, an unlabelled compound suspected of being a Fg homolog can be used to inhibit the binding of labelled Fg or a labelled Fg homolog to the ECR RGD-independent Fg-binding site of ECR on endothelial cells. The amount of labelled Fg binding to the binding site in the presence or absence of the unlabelled compound would be compared and if the presence of the unlabelled compound inhibits the amount of labelled Fg binding to the binding site, then the unlabelled compound is a Fg homolog. A preferred method for measuring inhibition of Fg binding to ECR is described in Example 5.

Another Fg homolog contemplated by the present invention are antibody molecules which immunoreact with the ECR RGD-independent Fg-binding site. Exemplary antibody molecules are anti-ECR antibodies and anti-ICAM-1 antibodies, defined further herein.

### 2. Endothelial Cell Receptor Homologs

An endothelial cell receptor (ECR) homolag, according to the present invention, is a macromolecule that mimics a region of ECR that binds to fibrinogen in an RGD-independent manner. The site on fibrinogen to which ECR binds in an RGD-independent manner is referred to as the "Fg RGD-independent ECR-binding site", that is, a site on Fg that binds to ECR in an RGD-independent manner.

An ECR homolog is any macromolecule which is capable of binding to the Fg RGD-independent ECR-binding site, and thereby can inhibit ECR binding to the Fg RGD-independent ECR-binding site on Fg, and thereby inhibit RGD-independent Fg binding to endothelial cells. Assays for measuring the binding of an ECR homolog to the Fg RGD-independent ECR-binding site are described in Example 3a. Assays for measuring the inhibition of ECR binding to the Fg RGD-independent ECR-binding site are described in Example 5.

The ECR homolog of the present invention is a substantially purified ICAM-1 homolog. A particularly preferred ECR homolog is ICAM-1 itself. Also contemplated ECR homologs are ICAM-1 associated or ICAM-1 related molecules.

An ECR homolog of the present invention may be modified, fragmented, coupled or otherwise manipulated as described herein for a Fg homolog so long as the desirable binding and inhibiting properties are maintained.

Particularly preferred are polypeptides and their analogues derived from the region of ICAM-1 that binds to fibrinogen, particularly a soluble ICAM-1 and functional derivatives thereof. A soluble ICAM-1 for use in a composition or method described herein typically lacks the transmembrane domain that serves as a membrane anchor. ICAM-1 and soluble ICAM-1 compositions suitable for use as an ECR homolog can be prepared by a variety of methods, including the purification methods described in Example 2. Additional preparative methods for ICAM-1 and soluble ICAM-1 are described in the Published EPO Application No. EP 365837 and Published Canadian Application No. 2008368.

Another ECR homolog contemplated by the present invention is an antibody molecule which immunoreacts with the Fg RGD-independent ECR-binding site. Exemplary antibody molecules are anti-Fg antibodies, defined further herein.

A Fg or ECR homolog can be coupled to or conjugated with another protein or polypeptide to produce a homolog conjugate. A homolog conjugate has advantages over a homolog used alone. For example, coupling the homolog to a protein or polypeptide known to contain a second biological function allows the targeting of that second biological function to the location at or near an ECR.

When coupled to a carrier to form what is known in the art as a carrier-hapten conjugate, a homolog of the present invention is capable of inducing antibodies that immunoreact with the homolog. In view of the well established principle of immunologic cross-reactivity, the present invention therefore contemplates antigenically related variants of a Fg or ECR homolog. An "antigenically related variant" is a subject polypeptide that is capable of inducing antibody molecules that immunoreact with a Fg homolog of this invention and with Fg, or with an ECR homolog of this invention and with ECR.

Any peptide of the present invention may be used in the form of a pharmaceutically acceptable salt. Suitable acids which are capable of forming-salts with the peptides of the present invention include inorganic acids such as hydrochloric acid, hydrobromic acid, perchloric acid, nitric acid, thiocyanic acid, sulfuric acid, phosphoric acetic acid, propionic acid, glycolic acid, lactic acid, pyruvic acid, oxalic acid, malonic acid, succinic acid, maleic acid, fumaric acid, anthranilic acid, cinnamic acid, naphthalene sulfonic acid, sulfanilic acid or the like.

Suitable bases capable of forming salts with the peptides of the present invention include inorganic bases such as sodium hydroxide, ammonium hydroxide, potassium hydroxide and the like; and organic bases such as mono-, di- and tri-alkyl and aryl amines (e.g. triethylamine, diisopropyl amine, methyl amine, dimethyl amine and the like) and optionally substituted ethanolamines (e.g. ethanolamine, diethanolamine and the like).

In other preferred embodiments a homolog is conjugated with a carrier molecule to form a homolog conjugate containing at least one carrier molecule. Typical carriers include Sepharose™, Sephadex™, proteins, polypeptides and the like.

A homolog may also be conjugated to itself or aggregated in such a way as to produce a large complex containing a homolog. A large complex containing a homolog is advantageous because it has new biologic properties such as longer half-life in circulation or greater activity.

### C. Compositions Containing Homologs

In one preferred embodiment, the invention contemplates a composition comprising a carrier and a therapeutically effective amount of a substantially pure and pharmaceutically acceptable fibrinogen (Fg) homolog according to this invention capable of binding to the ECR RGD-independent Fg-binding site and inhibiting fibrinogen binding to the ECR RGD-independent Fg-binding site. Preferred Fg homologs for use in a composition were described earlier.

In a related embodiment, the invention contemplates a composition comprising a carrier and a therapeutically effective amount of a substantially pure and pharmaceutically acceptable endothelial cell receptor homolog according to this invention capable of binding to the Fg RGD-independent ECR-binding site and inhibiting ECR binding to the Fg RGD-independent ECR-binding site. Preferred ECR homologs, particularly ICAM-1 homologs, for use is a composition were described earlier.

Both of these compositions are useful for inhibiting the binding of Fg to endothelial cells, thereby inhibiting endothelial cell/fibrinogen-mediated inflammation, and the associated disease processes described in more detail elsewhere herein.

A therapeutically effective amount of a Fg or ECR homolog is an amount that when administered to a patient is capable of inhibiting fibrinogen binding to endothelial cells. Assays for detecting the inhibition of Fg binding to endothelial cells and thereby measuring effective inhibiting amounts of homolog include, but are not limited to the competitive and other binding assays described in Example 5 of this specification.

Preferably, a therapeutically effective amount of a Fg or ECR homolog is an amount that reduces (inhibits) fibrinogen binding to endothelial cells by at least 10 percent, preferably by at least 50 percent, and more preferably by at least 99 percent, when measured in an in vitro assay for fibrinogen binding to endothelial cells. An exemplary in vitro assay to quantitate effective inhibitory amounts of a Fg homolog is described in Example 5.

In one embodiment, a therapeutic composition is useful for inhibiting endothelial cell/fibrinogen mediated inflammation in a patient exhibiting one or more of the conditions associated with inflammation as described further herein. In this embodiment, a therapeutically effective amount is an amount that when administered to a patient is sufficient to inhibiting fibrinogen binding to endothelial cell, thereby inhibiting endothelial cell/fibrinogen mediated inflammation.

Assays for directly detecting the inhibition of endothelial cell/fibrinogen mediated inflammation include, but are not limited to, clinical inspection of symptoms attendant in a patient presenting with inflammation.

Substantially pure, when used in the context of a Fg or ECR homolog, refers to compositions that are enriched in Fg or ECR homolog, and preferably are free of detectable amounts of blood cells, immunoglobulin and albumin proteins, and lipoproteins, and more preferably contains in excess of 99 percent by weight of homolog per total mass in the composition.

In one embodiment, the present invention contemplates compositions, and their methods of use, comprising both an Fg and ECR homolog of this invention in a range of ratios of Fg homolog to ECR homolog. The ratio can be anywhere from vast excesses of Fg homolog relative to ECR homolog, to vast excesses of ECR homolog to Fg homolog, ie, about 0.01:99.00 percent by weight. Preferred ratios range from 10:1 to 1:1. The only impermissible combination of Fg homologs and ECR homologs in a contemplated composition is the admixture of an anti-ECR antibody (Fg homolog) with an ECR homolog, or the admixture of an anti-Fg antibody (ECR homolog) with a Fg homolog, because these particular combinations will cross-immunoreact and neutralize effectiveness.

By pharmaceutically acceptable is meant that a Fg or ECR homolog, when used in a therapeutic composition, does not cause any undesirable physiological effects due to the presence of contaminants. Thus a pharmaceutically acceptable Fg or ECR homolog is free of pharmaceutically unacceptable contaminants such as pyrogens (lipopolysaccharides) and other contaminants such as poisonous chemicals (i.e., sodium azide) and detergents, namely sodium dodecyl sulfate.

The preparation of therapeutic compositions which contain polypeptides or proteins as active ingredients is well understood in the art. Typically, such compositions are prepared as injectables, either as liquid solutions or suspensions, however, solid forms suitable for solution in, or suspension in, liquid prior to injection can also be prepared. The preparation can also be emulsified. The active therapeutic ingredient is mixed with inorganic and/or organic carriers which are pharmaceutically acceptable and compatible with the active ingredient. Carriers are pharmaceutically acceptable excipients (vehicles) comprising more or less inert substances when added to a therapeutic composition to confer suitable consistency or form to the composition. Suitable carriers are, for example, water, saline, dextrose, glycerol, ethanol, or the like and combinations thereof. In addition, if desired, the composition can contain minor amounts of auxiliary substances such as wetting or emulsifying agents and pH buffering agents which enhance the effectiveness of the active ingredient.

A therapeutic composition useful in the practice of the present invention typically contains a Fg or ECR homolog formulated into the therapeutic composition as a neutralized pharmaceutically acceptable salt form. Pharmaceutically acceptable salts include the acid addition salts (formed with the free amino groups of the polypeptide or antibody molecule) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

The therapeutic Fg or ECR homolog-containing composition is conventionally administered parenterally, as by injection of a unit dose, for example. In this way the therapeutic composition can be delivered by a variety of means including intravenous, intramuscular, infusion, oral, intranasal, intraperitoneal, subcutaneous, rectal, topical, or into other regions, such as into synovial fluids. However delivery of a Fg or ECR homolog-containing composition transdermally is also contemplated, such by diffusion via a transdermal patch.

The term "unit dose" when used in reference to a therapeutic composition used in the present invention refers to physically discrete units suitable as unitary dosages for humans, each unit containing a predetermined quantity of active material calculated to produce the desired therapeutic effect in association with the required diluent, carrier or excipient.

In preferred embodiments, a therapeutic composition of the present invention contains an effective amount of a Fg or ECR homolog and is a sterile composition. A sterile composition is well understood in the pharmaceutical arts and is substantially free of bacteria and fungus. Typically a composition is made sterile by passing the composition through a filter such as a 0.2 micron filter designed for this purpose.

In other preferred embodiments, a composition of the present invention is optimized to allow the Fg or ECR homolog it contains to be delivered transdermally.

In other preferred embodiments, a composition of the present invention contains an immunologically effective amount of Fg or ECR homolog. An immunologically effective amount of Fg or ECR homolog is an amount sufficient to produce antibodies that immunoreact with the immunized homolog.

### D. Antibodies and Monoclonal Antibodies

The term "antibody" in its various grammatical forms is used herein as a collective noun that refers to a population of immunoglobulin molecules and/or immunologically active portions of immunoglobulin molecules, i.e., molecules that contain an antibody combining site or paratope.

An "antibody combining site" is that structural portion of an antibody molecule comprised of the heavy and light chain variable and hypervariable regions that specifically binds antigen. The site can be reduced to a minimum amount of the complementarity determining regions (CDRs) of the variable regions so long as the antigen-binding property is preserved.

The phrase "antibody molecule" in its various grammatical forms as used herein therefore contemplates both an intact immunoglobulin molecule and an immunologically active portion of an immunoglobulin molecule.

Exemplary antibody molecules for use in the methods and systems of the present invention are intact immunoglobulin molecules, substantially intact immunoglobulin molecules and those portions of an immunoglobulin molecule that contain the paratope, including those portions known in the art as Fab, Fab', F(ab')₂, F(v), and portions thereof.

Fab and F(ab')₂ portions of antibodies can be prepared by the proteolytic reaction of papain and pepsin, respectively, on substantially intact antibodies by methods that are well known. See for example, U.S. Patent No. 4,342,566, Inbar et al., Proc. Natl. Acad. Sci. USA, 69:2659-62 (1972), and Goding, Monoclonal Antibodies: Principles and Practice, Academic Press, P118-124 (1983).

Fab' antibody portions are also well known and are produced from F(ab')₂ portions followed by reduction of the disulfide bonds linking the two heavy reduction of the disulfide bonds linking the two heavy chain portions as with mercaptoethanol, and followed by alkylation of the resulting protein mercaptan with a reagent such as iodoacetamide. An antibody containing intact antibody molecules are preferred, and are utilized as illustrative herein.

### 1. Anti-ECR Homolog Antibodies

In one embodiment, the present invention contemplates an anti-ECR homolog antibody, ie, a composition comprising antibody molecules, that immunoreacts with an ECR homolog of this invention at or near the ECR RGD-independent Fg-binding site. Stated differently, and anti-ECR homolog antibody immunoreacts with ECR, is specific for the Fg-binding site on ECR as defined herein, and preferentially inhibits fibrinogen binding to stimulated endothelial cells.

A preferred anti-ECR antibody does not immunoreact with vitronectin receptor (VnR), ie, is substantially free of antibody molecules that immunoreact with VnR. More preferably, the antibody does not immunoreact with any RGD-dependent endothelial cell surface receptor, or with transglutaminase or the 130 kilodalton receptor which binds fibrin peptides.

By "preferentially inhibits" is meant that the antibody exhibits more inhibition of Fg binding to endothelial cells that are stimulated than endothelial cells that are not stimulated when assayed on endothelial cells in monolayer, such as is described in Example 5. In other words, a preferred antibody inhibits Fg binding to stimulated endothelial at lower antibody doses or resulting in lower Fg binding than the same antibody when tested on non-stimulated endothelial cells. Endothelial cells may be stimulated by a variety of means, including by exposure to cytokines such as TNF and chemoattractant peptides such as N-FMLP.

By "substantially free" means that the antibody molecules do not immunoreact with the stated antigen at levels within one order of magnitude, and preferably within two orders of magnitude, of the level of immunoreaction with a species of antigen recited to immunoreact with the antibody molecule when immunoreaction is expressed as an equilibrium constant between bound (immunoreacted) and nonbound antigen.

Antibody reactivity with a stated antigen can be measured by a variety of immunological assays known in the art. Exemplary immunoreaction assays are described herein.

The preparation of antibodies is well known in the art. See, Staudt et al., J. Exp, Med., 157:687-704 (1983), Examples 4 and 6 of the specification or Antibodies: A Laboratory Manual, Harlowe and Lane, Eds., Cold Spring Harbor, NY (1988). Briefly, to produce an antibody composition of this invention, a laboratory mammal is inoculated with an immunologically effective amount of a ECR homolog, typically as present in a vaccine or inoculum of the present invention, thereby inducing in the mammal antibody molecules having immunospecificity for the immunogen. The antibody molecules induced are then collected from the mammal and are isolated to the extent desired by well known techniques such as, for example, by immunoaffinity chromatography, or by using DEAE Sephadex™ to obtain the IgG fraction.

To enhance the specificity of the antibody, the antibody molecules are preferably purified by immunoaffinity chromatography using solid phase-affixed immunogen. The antibody is contacted with the solid phase-affixed immunogen for a period of time sufficient for the immunogen to immunoreact with the antibody molecules to form a solid phase-affixed immunocomplex. The bound antibodies are separated from the complex by standard techniques.

A vaccine useful for preparing antibodies of the present invention comprises immunologically effective amounts of both an immunogen and an immunopotentiator suitable for use in mammals.

An immunopotentiator is a molecular entity that stimulates maturation, differentiation and function of B and/or T lymphocytes. Immunopotentiators are well known in the art and include T cell stimulating polypeptides such as those described in U.S. Patent No. 4,426,324 and the C8-substituted guanine nucleosides described by Goodman et al., J. Immunol., 135:3284-88 (1985) and U.S. Patent No. 4,643,992.

The word "inoculum" in its various grammatical forms is used herein to describe a composition containing an ECR homolog of this invention as an active ingredient used for the preparation of antibodies of this invention.

When a small molecule such as a polypeptide is used in an inoculum to induce antibodies it is to be understood that the polypeptide can be used in various embodiments, e.g., alone or linked to a carrier as a conjugate, or as a polypeptide polymer. However, for ease of expression and in context of a polypeptide inoculum, the various embodiments of the polypeptides of this invention are collectively referred to herein by the term "polypeptide" and its various grammatical forms.

For a polypeptide that contains fewer than about 35 amino acid residues, it is preferable to use the peptide bound to a carrier for the purpose of inducing the production of antibodies.

One or more additional amino acid residues can be added to the amino- or carboxy-termini of the polypeptide to assist in binding the polypeptide to a carrier. Cysteine residues added at the amino- or carboxy-termini of the polypeptide have been found to be particularly useful for forming conjugates via disulfide bonds. However, other methods well known in the art for preparing conjugates can also be used.

The techniques of polypeptide conjugation or coupling through activated functional groups-presently known in the art are particularly applicable. See, for example, Aurameas, et al., Scand. J. Immunol., Vol. 8, Suppl. 7:7-23 (1978) and U.S. Patent No. 4,493,795, No. 3,791,932 and No. 3,839,153. In addition, a site directed coupling reaction can be carried out so that any loss of activity due to polypeptide orientation after coupling can be minimized. See, for example, Rodwell et al., Biotech., 3:889-894 (1985), and U.S. Patent No. 4,671,958.

Exemplary additional linking procedures include the use of Michael addition reaction products, di-aldehydes such as glutaraldehyde, Klipstein, et al., J. Infect. Dis., 147:318-326 (1983) and the like, or the use of carbodiimide technology as in the use of a water-soluble carbodiimide to form amide links to the carrier. Alternatively, the heterobifunctional crosslinker SPDP (N-succinimidyl-3-(2-pyridyldithio) proprionate)) can be used to conjugate peptides, in which a carboxy-terminal cysteine has been introduced.

Useful carriers are well known in the art, and are generally proteins themselves. Exemplary of such carriers are keyhole limpet hemocyanin (KLH), edestin, thyroglobulin, albumins such as bovine serum albumin (BSA) or human serum albumin (HSA), red blood cells such as sheep erythrocytes (SRBC), tetanus toxoid, cholera toxoid as well as polyamino acids such as poly (D-lysine: D-glutamic acid), and the like.

The choice of carrier is more dependent upon the ultimate use of the inoculum and is based upon criteria not particularly involved in the present invention. For example, a carrier that does not generate an untoward reaction in the particular animal to be inoculated should be selected.

The present inoculum contains an effective, immunogenic amount of a homolog of this invention, typically as a conjugate linked to a carrier. The effective amount of homolog per unit dose sufficient to induce an immune response to the immunogen depends, among other things, on the species of animal inoculated, the body weight of the animal and the chosen inoculation regimen as is well known in the art. Inocula typically contain homolog concentrations of about 10 micrograms to about 500 milligrams per inoculation (dose), preferably about 50 micrograms to about 50 milligrams per dose.

The term "unit dose" as it pertains to the inocula refers to physically discrete units suitable as unitary dosages for animals, each unit containing a predetermined quantity of active material calculated to produce the desired immunogenic effect in association with the required diluent; i.e., carrier, or vehicle. The specifications for the novel unit dose of an inoculum of this invention are dictated by and are directly dependent on (a) the unique characteristics of the active material and the particular immunologic effect to be achieved, and (b) the limitations inherent in the art of compounding such active material for immunologic use in animals, as disclosed in detail herein, these being features of the present invention.

Inocula are typically prepared from the dried solid homolog-conjugate by dispersing the conjugate in a physiologically tolerable (acceptable) diluent such as water, saline or phosphate-buffered saline to form an aqueous composition.

Inocula can also include an adjuvant as part of the diluent. Adjuvants such as complete Freund's adjuvant (CFA), incomplete Freund's adjuvant (IFA) and alum are materials well known in the art, and are available commercially from several sources.

The anti-homolog specific antibody so produced can be used, inter alia, in the therapeutic and diagnostic methods and systems of the present invention to inhibit fibrinogen binding to endothelial cells, and to detect homologs present in a sample such as a body fluid sample.

An antibody of this invention is preferably a monoclonal antibody due to the controlled specificity offered by a monoclonal antibody.

The phrase "monoclonal antibody" in its various grammatical forms refers to a population of antibody molecules that contain only one species of antibody combining site capable of immunoreacting with a particular epitope. A monoclonal antibody thus typically displays a single binding affinity for any epitope with which it immunoreacts. A monoclonal antibody may therefore contain an antibody molecule having a plurality of antibody combining sites, each immunospecific for a different epitope, e.g., a bispecific monoclonal antibody.

A monoclonal antibody is typically composed of antibodies produced by clones of a single cell called a hybridoma that secretes (produces) only one kind of antibody molecule. The hybridoma cell is formed by fusing an antibody-producing cell and a myeloma or other self-perpetuating cell line.

The preparation of such antibodies was first described by Kohler and Milstein, Nature 256:495-497 (1975). An exemplary hybridoma technology is described by Niman et al., Proc. Natl. Acad. Sci., U.S.A., 80:4949-4953 (1983). Other methods of producing a monoclonal antibody, a hybridoma cell, or a hybridoma cell culture are also well known. See, for example, Antibodies: A Laboratory Manual, Harlow et al., Cold Spring Harbor Laboratory, 1988; or the method of isolating monoclonal antibodies from an immunological repertoire as described by Sastry, et al., Proc. Natl. Acad. Sci. USA, 86:5728-5732 (1989); and Huse et al., Science, 246:1275-1281 (1981). The references cited are hereby incorporated herein by reference.

The hybridoma so prepared produces a supernate that can be screened for the presence of antibody molecules that immunoreact with a homolog of this invention, or for inhibition of fibrinogen binding to endothelial cells as described further herein.

Briefly, to form the hybridoma from which the monoclonal antibody composition is produced, a myeloma or other self-perpetuating cell line is fused with lymphocytes obtained from the spleen of a mammal hyperimmunized with a homolog of this invention as the immunogen.

It is preferred that the myeloma cell line used to prepare a hybridoma be from the same species as the lymphocytes. Typically, a mouse of the strain 129 GlX⁺ is the preferred mammal. Suitable mouse myelomas for use in the present invention include the hypoxanthine-aminopterin-thymidine-sensitive (HAT) cell lines P3X63-Ag8.653, and Sp2/0-Ag14 that are available from the American Type Culture Collection, Rockville, MD, under the designations CRL 1580 and CRL 1581, respectively.

Splenocytes are typically fused with myeloma cells using polyethylene glycol (PEG) 1500. Fused hybrids are selected by their sensitivity to HAT. Hybridomas producing a monoclonal antibody of this invention are identified using the enzyme linked immunosorbent assay (ELISA) described in Example 4.

A monoclonal antibody of the present invention can also be produced by initiating a monoclonal hybridoma culture comprising a nutrient medium containing a hybridoma that produces and secretes antibody molecules of the appropriate polypeptide specificity. The culture is maintained under conditions and for a time period sufficient for the hybridoma to secrete the antibody molecules into the medium. The antibody-containing medium is then collected. The antibody molecules can then be further isolated by well known techniques.

Media useful for the preparation of these compositions are both well known in the art and commercially available and include synthetic culture media, inbred mice and the like. An exemplary synthetic medium is Dulbecco's minimal essential medium (DMEM; Dulbecco et al., Virol. 8:396 (1959)) supplemented with 4.5 gm/l glucose, 20 mm glutamine, and 20% fetal calf serum. An exemplary inbred mouse strain is the Balb/c.

The monoclonal antibodies of this invention can be used in the same manner as disclosed herein for antibodies of the present invention.

For example, the monoclonal antibody can be used in the therapeutic, diagnostic or in vitro methods disclosed herein where inhibition of fibrinogen binding to endothelial cells is desired.

A preferred monoclonal antibody immunoreacts with the prototype ECR described herein, namely ICAM-1. The anti-ICAM-1 monoclonal antibody was produced by immunization with endothelial cells, followed by screening for the ability to inhibit Fg binding to endothelial cells, as described in Example 4.

A particularly preferred anti-ICAM-1 monoclonal antibody is the monoclonal antibody produced by the hybridoma 14E11, 16G8, 2E12, and 2B12 that immunoreact with ICAM-1, and inhibit Fg binding to endothelial cells.

Also contemplated are monoclonal antibodies having a binding specificity for the same or cross-reacting epitopes,ie, immunospecific for the same epitope, on ICAM-1 as the above preferred anti-ICAM-1 antibodies, or derived from the above antibodies. Thus, the present invention contemplates a monoclonal antibody, and immunoreactive fragments thereof, that has the immunospecificity of a monoclonal antibody produced by a hybridoma selected from the group consisting of 14E11, 16G8, 2E12, and 2B12.

Immunological techniques for determining the immunospecificity of a monoclonal antibody are well known in the art, and can include competition binding studies and other cross-reaction assays. See for example the immunoassays described in Antibodies: A Laboratory Manual, Harlow et al., Cold Spring Harbor Laboratory, 1988.

Also contemplated by this invention is the hybridoma cell, and cultures containing a hybridoma cell that produce a monoclonal antibody of this invention.

Hybridomas 14E11, 16G8, 2E12, and 2B12 have been deposited pursuant to Budapest Treaty requirements with the American Type Culture Collection (ATCC), Rockville, MD, on June 10, 1992, and were assigned accession numbers HB 11064, 11063, 11061 and 11062, respectively.

Hybridomas 14E11, 16G8, 2E12, and 2B12 were deposited in a depository affording permanence of the deposit and ready accessibility thereto by the public upon the issuance of a patent, under conditions which assure that access to the hybridomas will be available during the pending of the patent application to those deemed by the Commissioner to be entitled to such access, and that all restrictions on the availability to the public of the hybridomas as deposited will be irrevocably removed upon the granting of the patent. The deposited hybridomas will be maintained by the ATCC for the term of the patent or 30 years from the date of deposit, whichever is longer, and in all events for at least five years after the date of the last request for access.

### E. Methods of Inhibiting Fibrinogen Binding To Endothelial Cells and Inhibiting Fibrinogen/Endothelial Cell-Mediated Inflammation

The present invention contemplates a method of inhibiting fibrinogen (Fg) binding to endothelial cells by contacting said endothelial cells with a therapeutically effective amount of a composition containing a Fg or ECR homolog, or both, of this invention dispersed in a pharmaceutically acceptable excipient.

As described herein, the use of a Fg homolog or an ECR homolog, or both, exhibit(s) therapeutically effective inhibition of Fg binding to endothelial cells because these two therapeutic reagents mimic, as homologs, their natural counterparts and thereby block the fibrinogen-ECR interaction as identified by the present invention.

In the examples herein, the ECR homolog monoclonal antibody anti-ICAM-1 will be used as an exemplary therapeutic reagent for use in a composition for the present method. In another example, the Fg homolog, intact fibrinogen, is used as an exemplary therapeutic reagent. However, it should be understood that the invention contemplates the use of any Fg or ECR homolog and is not limited to those specific reagents.

Insofar as the binding of Fg to endothelial cells mediates fibrinogen and endothelial cell-mediated inflammation, inhibiting Fg binding in vivo provides a method for inhibiting inflammation in a patient suffering from or at risk for fibrinogen and endothelial cell-mediated inflammation.

Thus, the present invention also contemplates a method of inhibiting fibrinogen/endothelial cell-mediated inflammation in a patient comprising administering to the patient a therapeutically effective amount of a pharmaceutically acceptable composition comprising a substantially pure homolog selected from the group consisting of a Fg homolog and an ICAM-1 homolog dispersed together with a pharmaceutically acceptable excipient (carrier).

Patients in which the inhibition of Fg binding to endothelial cells, and the inhibition of inflammation, would be clinically useful include patients with various types of inflammation, or at risk of inflammation, including but not limited to patients with very recent myocardial infarction (within 40 hours of the acute event) where the Fg or ECR homolog would prevent neutrophil accumulation on exposed tissues due to injury to those tissues, patients with autoimmune responses, general inflammatory or localized inflammatory reactions, glomerular nephritis, delayed type hypersensitivity, psoriasis, autoimmune thyroiditis, multiple sclerosis, rheumatoid arthritis, lupus erythematosis, tissue transplants, graft rejection, reperfusion injury of tissue, and the like inflammatory disorders.

The inhibition of fibrinogen/endothelial cell-mediated inflammation can be detected by measuring changes in the amount of neutrophil accumulation at the site of an inflammation producing injury or wound. For example, the number of neutrophils that accumulate at the site of a sponge placed under the skin can be determined both before and after a Fg or ECR homolog is administered to the patient. See, for example, Price et al., J. Immunol., 139:4174-4177 (1987).

Fibrinogen/endothelial cell-mediated inflammation includes any of the various biological processes mediated lymphocyte having a Mac-1 receptor on its cell surface. Typical biological processes include adhesion of Mac-1 bearing cells to vascular endothelium and specific interactions with extracellular matrix proteins.

A homolog is typically administered as a pharmaceutically acceptable composition in the form of a solution or suspension. However, as is well known, peptides and proteins such as a Fg or ECR homolog can also be formulated for therapeutic administration as tablets, pills, capsules, sustained release formulations or powders. Typically, suitable dosage ranges for an therapeutic composition are of the order of one to hundreds of nanomoles of Fg or ECR homolog per kilogram body weight per minute and depend on the route of administration. In any case, the administered composition contains at least about 0.10% to about 99% by weight of a Fg or ECR homolog per weight of composition, preferably 10%-90% and more preferably 25-75%.

The composition is administered in a manner compatible with the dosage formulation, and in a therapeutically effective amount. The quantity to be administered depends on the subject to be treated, capacity of the subject's blood hemostatic system to utilize the active ingredient, and degree of inflammation inhibition or fibrinogen binding inhibition desired. Precise amounts of active ingredient required to be administered depend on the judgement of the practitioner and are peculiar to each individual.

A therapeutically effective amount of homolog can be expressed as an amount sufficient to produce a final concentration of homolog in a patient's blood. That blood concentration can be determined by an in vitro assay for the homolog in a liquid body sample (e.g., blood), such as is described herein, or can be calculated based on the patient's body weight and blood volume as is well known.

Suitable dosage ranges of a homolog for the therapeutic methods described herein are in the order of about 0.1 to about 20 milligrams, preferably one to ten milligrams of homolog per kilogram of body weight of patient per day, and depending on the route of administration. Stated differently, a therapeutically effective dosage is an amount sufficient to produce an intravascular concentration of in the blood of the patient in the range of about 0.1 to about 100 micrograms/milliliter (*µ*g/ml), preferably about 10 to about 20 *µ*g/ml of the active ingredient.

### F. Methods of Detecting Homolog

The present invention contemplates any method that results in detecting a homolog by producing a reaction product using a monoclonal antibody, polyclonal antibody, or homolog binding reagent.

Due to the binding interaction of a Fg homolog and an ECR homolog, a Fg homolog binding reagent can be any ECR homolog, and an ECR homolog binding reagent can be any Fg homolog.

Those skilled in the art will understand that there are numerous well known clinical diagnostic chemistry procedures that can be utilized to form and detect such reaction products. Thus, while exemplary assay methods are described herein, the invention is not so limited.

Various heterogeneous and homogeneous assay protocols can be employed, either competitive or non-competitive for detecting the presence and preferably the amount of a Fg or ECR homolog in a tissue or liquid composition.

A Fg or ECR homolog may be detected in any sample such as a solid, liquid or body fluid sample. In preferred embodiments a homolog is detected in body fluid samples include blood, plasma, serum, mucous, sputum and the like.

A homolog may also be detected in vitro or in vivo in various tissues and organs. In preferred embodiments tissue slices or tissue sections may be assayed for the presence and location of a homolog. In other preferred embodiments organs may be assayed in vivo for the presence and to determine the location of a homolog.

Detection of the amount of homolog present in vitro or in vivo is useful because the amount of homolog present correlates with the progress of therapeutically administered homolog present in the patient being analyzed. Thus determination of the amount of homolog present in the patient being analyzed allows the therapeutic administration of a homolog to a patient to be monitored to determine the clinical state of the patient.

In one embodiment the present invention contemplates a method of detecting the presence and preferably the amount, of a Fg homolog in a liquid composition. The steps of this method include:
(1) admixing a sample of endothelial cells with a predetermined amount of a liquid sample containing a Fg homolog and a predetermined amount of labelled Fg homolog to form a competition reaction admixture;
(2) maintaining the reaction admixture formed in step (1) for a predetermined time period sufficient for the Fg homolog present in the liquid composition to bind to the endothelial cells and form a endothelial cell:Fg homolog complex and to allow the labelled Fg homolog to bind the endothelial cells and form a labelled endothelial cell:Fg homolog complex;
(3) assaying for the presence and/or amount of labelled endothelial cell:Fg homolog complex formed in step (2) thereby detecting the presence and/or amount of a Fg homolog in the composition.

A predetermined amount of a liquid composition containing a Fg homolog is a known amount of Fg-containing liquid composition that is useful and easily assayed. This predetermined amount of Fg homolog containing liquid composition has been shown to be useful by performing a series of test assays with an amount of liquid composition containing a known concentration of Fg homolog and is sufficient to allow the assay to be performed. Preferred amounts of a liquid composition are from about 1 microliter (*µ*l) to about 1000 *µ*l.

The liquid composition also contains a labelled Fg homolog. A label is an atom or molecule that is either directly or indirectly involved in the production of a detectable signal to indicate the presence of a complex. Any label or indicating means can be linked to or incorporated in an expressed protein, polypeptide, or antibody or monoclonal antibody composition of the present invention, or used separately, and those atoms or molecules can be used alone or in conjunction with additional reagents. Labels include various in vivo labels useful within the body of a patient such as ¹¹¹In, ⁹⁹Tc, ⁶⁷Ga, ¹⁸⁶Re, and ¹³²I.

The label can be a fluorescent labelling agent that chemically binds to antibodies or antigens without denaturing them to form a fluorescent (dye) that is a useful immunofluorescent tracer. Suitable fluorescent labelling agents are fluorochromes such as fluorescein isocyanate (FIC), fluorescein isothiocyanate (FITC), 5-dimenthylamin-1-naphthalenesulfonyl chloride (DANSC), tetramethylrhodamine isothiocyanate (TRITC), lissamine, rhodamine 8200 sulphonyl chloride (RB 200 SC) and the like. A description of immunofluorescence analysis techniques in found in Deluca, "Immunofluorescence Analysis", in Antibody As A Tool, Marchalonis et al., eds., John Wiley & Sons, Ltd., pp. 189-231 (1982), which is incorporated herein by reference.

In preferred embodiments the label is an enzyme, such as horseradish peroxidase (HRP), glucose oxidase, or the like. In such cases where the principal indicating group is an enzyme such as HRP or glucose oxidase, additional reagents are required to visualize the fact that a receptor-ligand complex (immunoreactant) has formed. Such additional reagents for HRP include hydrogen peroxide and an oxidation dye precursor such as diaminobenzidine. An additional reagent useful with glucose oxidase is 2,2'-azino-di(3-ethyl-4-2-benzthiazoline-G-sulfonic acid) (ABTS).

Radioactive elements are also useful as labels. An exemplary radiolabel is a radioactive element that produces gamma ray emissions. Elements which themselves emit gamma rays, such as ¹²⁴I, ¹²⁵I, ¹²⁸I, ¹³¹I, ¹³²I, and ⁵¹Cr represent one class of gamma ray emission-producing radioactive element indicating groups. Particularly preferred is ¹²⁵I. Another group of useful indicating groups are those elements such as ¹¹C, ¹⁸F, ¹⁵O and ¹³N which themselves emit positrons. The positrons so emitted produce gamma rays upon encounters with electrons present in the animal's body. Also useful is a beta emitter, such as indium.

The linking of labels, i.e., labelling of, polypeptides and proteins such as a Fg homolog is well known in the art. For instance, antibody molecules produced by a hybridoma can be labelled by metabolic incorporation of radioisotope-containing amino acids provided as a component in the culture medium. See, for example, Galfre et al., Meth. Enzymol., 73:3-46 (1981). The techniques of protein conjugation or coupling through activated functional groups are particularly applicable. See, for example, Aurameas et al., Scand. J. Immunol., Vol. 8, 7:7-23 (1978), Rodwell et al., Biotech., 3:889-894 (1985), and U.S. Patent No. 4,493,795.

In addition, site directed coupling reactions can be carried out so that the label does not substantially interfere with the ability of the antibody molecules to bind their specific antigen. See, for example, Rodwell et al., Biotech., 3:889-894 (1985).

The reaction admixture is maintained for a predetermined time period sufficient for the Fg homolog and the labelled Fg homolog present in the liquid composition to bind to the endothelial cells and form an endothelial cell:Fg homolog complex and a labelled endothelial cell:Fg homolog complex.

The amount of time sufficient for the Fg homolog and the labelled Fg homolog to bind the endothelial cells depends upon several physical parameters including temperature and the concentration of the various reactants. In preferred embodiments, the predetermined time period is from about 1 minute to 24 hours. In more preferred embodiments the predetermined time period is from about 10 minutes to about 1 hour. In the most preferred embodiments, the predetermined time period is from about 15 minutes to 30 minutes. Typically this time period is predetermined to optimize the assay.

Typically the reaction admixture is maintained under biological assay conditions that maintain the activity of the polypeptide and protein molecules including the Fg homolog and the endothelial cell sought to be assayed, and include a temperature range of about 4 degrees C (4°C) to about 45°C, a pH value range of about 5 to about 9 and an ionic strength varying from that of distilled water to that of about one molar sodium chloride. Methods for optimizing such conditions are well know in the art.

The presence of labelled endothelial cell:Fg homolog complex formed by maintaining the reaction admixture in step (2) is assayed.

The direct or indirect methods used to assay for the presence of and preferably the amount of labelled endothelial cell:Fg homolog complex formed depend on the particular label used and are well known in the art. For example, the amount of radioactivity in the labelled endothelial cell:Fg homolog complex may be determined as described in Example 5. Alternatively, homogeneous assay methods such as those described in U.S. Patent No. 4,536,479; No. 4,233,401; No. 4,233,402 and No. 3,996,345.

In other preferred embodiments, the present invention contemplates another method of detecting the amount of a Fg homolog in a liquid sample using an Fg homolog binding reagent, ie, an ECR homolog. The steps of this method include;
(1) admixing an ECR homolog with a predetermined amount of a liquid sample containing a Fg homolog to form an binding reaction admixture;
(2) maintaining the binding reaction admixture formed in step (1) for a preselected time period sufficient for the Fg homolog present in the liquid sample to bind to the ECR homolog and form a complex containing Fg homolog and ECR homolog; and
(3) determining the amount of the complex formed in step (2), thereby detecting the amount of a Fg homolog within the liquid sample.

A preferred ECR homolog is an anti-Fg monoclonal antibody, and the complex formed is an immunoreaction complex.

In a related embodiment, the invention contemplates a method for the detection of the amount of an ECR homolog in a liquid sample using an ECR homolog binding reagent, ie, a Fg homolog. The method is practiced in the same manner as above, except that an Fg homolog is added as the binding reagent to a sample containing an ECR homolog.

Preferably, the liquid sample containing a homolog is a biological fluid sample such as blood, plasma, serum, sputum, saliva, and the like. Preferably, the amount of liquid sample admixed is known.

For the determining step, it is preferred that the added homolog (Fg homolog for detecting ECR homolog, and ECR homolog for detecting Fg homolog) is labelled, i.e., operatively linked to an indicating means such as an enzyme, radionuclide and the like as described earlier. In this embodiment, the determination is made by detecting the presence/amount of the label in the complex, thereby determining the presence/amount of the homolog in the sample.

In one embodiment, the added homolog is present as part of the solid support, i.e., operatively linked to a solid matrix, so that the reaction admixture formed is a solid and liquid phase, with the objective of "capturing" the sample to be determined.

The reaction admixture is maintained for a predetermined time period sufficient for the homolog present in the liquid sample to bind to the antibody and form a complex containing a homolog to be detected and the added homolog.

Biological assay conditions are those conditions that maintain the biological activity of the reagents and the homolog to be assayed as discussed earlier.

In a preferred embodiment, the amount of homolog in the complex can be determined, either directly or indirectly, using assay techniques well known in the art, and typically depend upon the type of indicating means used.

### G. Detection of ECR Receptors In Vivo

A method of detecting the presence and preferably the amount and location of cells having ECR receptors in a mammal is contemplated. An effective amount of a composition containing a physiologically tolerable diluent and an amount of Fg homolog linked to an in vivo indicating means is parenterally administered to a human subject. Parenteral administration includes intramuscular administration, intravenous administration, and administration into other body sites, such as synovial fluid. The amount of composition administered is sufficient to bind a detectable quantity of ECR receptors. In preferred embodiments the Fg homolog is anti-ICAM-1 antibody molecules, or D₃₀ fragment.

As used herein, the terms "label" and "indicating means" in their various grammatical forms refer to single atoms and molecules that are either directly or indirectly involved in the production of a detectable signal to indicate the presence of a complex. "In vivo" labels or indicating means are those useful within the body of a human subject. Any label or indicating means can be linked to or incorporated in an expressed protein, polypeptide, or antibody molecule that is part of an antibody or monoclonal antibody composition of the present invention, or used separately, and those atoms or molecules can be used alone or in conjunction with additional reagents. Such labels are themselves well known in clinical diagnostic chemistry and constitute a part of this invention only insofar as they are utilized with otherwise novel proteins methods and/or systems.

The linking labels, i.e., labelling of, polypeptides and proteins is well known in the art. For instance, antibody molecules produced by a hybridoma can be labelled by metabolic incorporation of radioisotope-containing amino acids provided as a component in the culture medium. See, for example, Galfre et al., Meth. Enzymol., 73:3-46 (1981). The techniques of protein conjugation or coupling through activated functional groups are particularly applicable. See, for example, Aurameas et al., Scand. J. Immunol., Vol. 8, 7:7-23 (1978), Rodwell et al., Biotech., 3:889-894 (1984) and U.S. Patent No. 4,493,795.

The subject is then maintained for a predetermined time period sufficient for the Fg homolog to bind to the ECR receptors present on the cells of the human subject and form a ECR:Fg homolog complex. Preferably, this time period has been predetermined to optimize the formation of an ECR:Fg homolog complex.

The subject is then assayed for the presence of and preferably the location of any ECR:Fg homolog complexes formed.

### H. Method For Identifying Inhibitors

The present invention also contemplates methods for identifying a composition that inhibits fibrinogen binding interaction to endothelial cells where the interaction is mediated by the fibrinogen binding site on ECR as described herein.

The method is generally useful for the design of novel therapeutics used in the inhibition of endothelial cell/fibrinogen mediated inflammation, and is particularly useful as a mass screening procedure to identify active inhibitor compounds and formulations.

The invention therefore contemplates a method for identifying a composition which inhibits fibrinogen binding to ECR on endothelial cells, which comprises:
(a) incubating components comprising the composition to be tested together with an ECR homolog and a Fg homolog under conditions which allow the ECR homolog to interact and bind with the Fg homolog; and
(b) measuring the interaction of the ECR homolog with the Fg homolog, thereby measuring the capacity of the composition to inhibit the interaction.

A preferred ECR homolog is ICAM-1, and a preferred Fg homolog is Fg, as defined herein.

The measuring can be directed at detecting free Fg homolog, free ECR homolog, or free composition. Alternatively, the measuring can detect the binding interaction of the composition with either the ECR homolog or the Fg homolog. Typically, the binding interaction is measured by detecting a complex formed upon binding.

Conditions sufficient for a binding interaction are generally physiological, and are time temperature and buffer conditions compatible with the binding of fibrinogen onto endothelial cells, as shown in the

### Examples.

More preferably, the binding interaction is detected in assays where one or the other of Fg homolog and ECR homolog are in the solid phase, and the other is labelled. The measuring comprised detecting the presence, and preferably amount of label in the solid phase, directly indicating the amount of inhibition by the composition.

In a related embodiment, the invention describes a method of screening for compositions effective at inhibiting fibrinogen binding to ECR comprising the steps of:
a) admixing in an inhibition reaction admixture preselected amounts of a putative inhibitor composition, a fibrinogen homolog, and an ECR homolog as defined herein;
b) maintaining said admixture under conditions sufficient for said ECR homolog to bind to said Fg homolog and form an ECR homolog:Fg homolog complex; and
c) measuring the amount of ECR homolog:Fg homolog complex formed in step (b), and thereby the effectiveness of said inhibitor composition.

In practicing the method, one of the homologs is labelled and in the liquid phase, and the other homolog is in the solid phase, wherein the measuring involves detecting the amount of label in the solid phase. Other formats are readily apparent.

Preferably, the ECR homolog is purified ICAM-1. More preferably, the ECR homolog is in the solid phase. Still more preferably, the solid phase is a cell on which ECR is located such as an endothelial cell, lymphoid cell, or a recombinant cell capable of expressing recombinant ICAM-1.

Exemplary screening methods are described in Example 5 where antibodies where identified that inhibit Fg binding to endothelial cells. Fg homologs and ECR homologs can also be developed and/or identified by the above methods.

### Examples

The following examples are intended to illustrate, but not limit, the present invention.

### 1. Preparation of Fibrinogen Analogs

### A. Purification of Plasma Fibrinogen

Fibrinogen was isolated from fresh plasma by cold ethanol fractionation procedures. To one volume of plasma, 0.22 volumes of cold 50% ethanol, pH 7.0 was admixed which lowered the temperature to -3 degrees Celsius (-3°C). The admixture was centrifuged and the resultant precipitate was washed with 0.5 original volumes (OV) of 7% ethanol, pH 6.5 at -3°C. The precipitate was re-collected and dissolved in 0.25 0V of 0.55 M trisodium citrate buffer, pH 6.5 at 30°C. The resultant solution was cooled to 0°C and the fibrinogen was precipitated by the addition of cold 20% ethanol to a final concentration of 8% to form purified fibrinogen.

To remove any possible contamination of the purified fibrinogen with fibronectin, the purified fibrinogen preparation was passed over a gelatin Sepharose™ 4B column (Pharmacia LKB, Piscataway, NJ) according to manufacturer's instructions resulting in fibronectin-free fibrinogen.

### B. Preparation of D₃₀ from Purified Fibrinogen

### 1) Proteolytic Digestion of Purified Fibrinogen

Fifty milligrams (mg) of purified fibrinogen prepared in Example 1A was dissolved in 1 milliliter (ml) of a TBS buffer solution containing 0.01 M Tris(hydroxymethyl)aminomethane (Tris-HCl), 0.14 M sodium chloride (NaCl), pH 7.4, and was proteolytically digested by Streptokinase-activated plasminogen (plasmin) according to the following procedure.

Streptokinase-activated plasminogen was prepared by admixing plasminogen (KABI, 20 units (U)) to 2 ml of 0.1 M sodium phosphate buffer, pH 7.4 and pre-maintaining for 10 minutes at 37°C with 500 U of streptokinase (Streptase, Behring). This solution was then admixed at a final concentration of 18 micrograms per ml (*µ*g/ml) to the solution of purified fibrinogen in 2 M urea.

The admixture was maintained for 2 hours at 37°C. The proteolytic reaction in the admixture was terminated by the addition of 50,000 U/ml trasylol (Sigma Chemical Co., St. Louis, MO). The resulting solution of fibrinogen fragments was extensively dialyzed against a solution of TBS for 24 hours at 4°C. The dialysis buffer was changed every 8 hours. The dialyzed solution was then recovered and applied on a Sephadex™ G-100 column (Pharmacia LKB). The column chromatography was performed to separate the fragments resulting from the proteolytic digestion of fibrinogen. The column was prewashed with a running buffer of TBS followed by application of the dialyzed fibrinogen fragments. Fractions of 3 ml were collected and the molecular weights of the separated fragments in the fractions were determined by 10% sodium dodecyl sulfate-polyacrylamide gel electrophoresis (SDS-PAGE) with and without reduction by 3% mercaptoethanol.

Three fragments of different molecular weights were visualized by Coomassie Blue staining of the gel. Fragments X, D, and E had respective molecular weights of approximately 240,000, 85,000, and 50,000 under non-reducing conditions. The fractions corresponding to the three separate peaks were separately pooled, dialyzed against distilled water, and concentrated by lyophilization.

### 2) Proteolytic Digestion of Fragment D to Produce a D₃₀ Homolog

Fibrinogen fragment D with a molecular weight of 80,000 (80 kilodaltons (kD)), purified and concentrated, was proteolytically digested with plasmin in 2 M urea for 24 hours at 37°C as described in Example 1B. The digestion was terminated and the resultant solution dialyzed as described in Example 1B. The dialyzed solution was recovered and the products of the digestion were isolated by high performance liquid chromatography (HPLC) on a Mono-Q-column (Pharmacia LKB) equilibrated in 0.01 M sodium phosphate, pH 7.0. The fragments were eluted with a solution of 0.01 M sodium phosphate and 1 M sodium chloride, pH 7.0. The purity of the eluted proteins in the collected fractions was assayed on 15% SDS-PAGE under non-reducing conditions. Coomassie Blue staining of the gel revealed a 30 kD fragment of greater than 90% homogeneity. The purified proteolytic digestion product of fragment D having a kD of 30 was designated D₃₀. The peak fractions containing D₃₀ were pooled and concentrated by lyophilization.

### 2. Purification of the Endothelial Cell RGD-Independent Fibrinogen Receptor

### A. Preparation of an Human Umbilical Vein Endothelial Cell Lysate

Human umbilical vein endothelial cells, (HUVEC) commercially available from Clonetics, San Diego, CA, were passaged into 40 gelatin-coated T75 tissue culture flasks (Falcon, Thousand Oaks, CA) and maintained in endotoxin-free RPMI 1640 (Whittaker M.A. Bioproducts, Walkersville, MD) supplemented with 10% fetal bovine serum (FBS) (Hyclone, Sterile Systems, Logan, UT), 25 mM Hepes [4-(2-hydroxyethyl)-1-piperidineethanesulfonic acid] (Calbiochem Boehring, La Jolla, CA), 100 *µ*g/ml penicillin-streptomycin-fungizone (Whittaker), 0.5% endothelial cell growth factor (Biomedical Technologies, Stoughton, MA) and 1 mM glutamine (Whittaker). In order to increase the yield of purified endothelial cell receptor (ECR), the cultured endothelial cells, at a density of approximately 5 X 10⁶ cells/flask, were stimulated 6 hours prior to harvesting by exposure to tumor necrosis factor alpha (TNF, Genzyme Corp., Cambridge, MA) at a concentration of 20 ng/ml. The cellular response to TNF was initially revealed in experiments described in Example 3A. After the culture medium was removed, the cells were detached from the flasks with 4 mM ethylenediaminetetraacetic acid (EDTA, Sigma Chemical Co.) at 37°C for 30 minutes. The detached cell suspensions from all the flasks were pooled and pelleted by centrifugation at 1200 rpm for 10 minutes. The pelleted cells were washed twice with cold phosphate-buffered saline (PBS).

After the final wash, the cells were resuspended in PBS containing 1 millimolar (mM) calcium chloride (CaCl₂) and 1 mM magnesium chloride (MgCl₂) in preparation of labelling cell surface proteins with 4 mCi of sodium ¹²⁵iodide (¹²⁵I) (NEN Du Pont de Nemours, Wilmington, DE) by the lactoperoxidase iodination method known to one skilled in the art and as described in "Antibodies: A Laboratory Method", Eds Harlow et al., Cold Spring Harbor Laboratory, pp 434-435 (1988). After the labelling procedure, the cells were washed 3 times with PBS lacking all cations. After the final centrifugation wash, the pellet was frozen, thawed then resuspended in a volume of 3 parts Tris-buffered saline extraction buffer (TBS extraction buffer) to 1 part pellet. TBS extraction buffer consisted of 25 mM Tris-HCl, 136 mM NaCl and 2 mM potassium chloride (KCl) that also contained 1-2 mM MgCl₂, 1-2 mM manganese chloride (MnCl₂), 50 mM octyl beta glucopyranoside, 1 mM phenylmethylsulfonyl-fluoride (PMSF), 1 *µ*g/ml aprotinin, 1 *µ*g/ml leupeptin, 1 *µ*g/ml pepstatin and 1 *µ*g/ml alpha 2 macroglobulin. The preferred concentrations of MgCl₂ and MnCl₂ were 1 mM. Calcium chloride was absent from the extraction buffer and all subsequent buffers used in the isolation and purification of the ECR.

The resultant cell lysate having 3 ml was centrifuged at 3000 X g to pellet the insoluble cellular debris. The supernatant containing the isolated ECR was removed and labelling efficiency was determined by gamma detection. Approximately a specific activity of 200,000 counts per minute (cpm) per 10 microliters (*µ*l) was obtained by the labelling procedure.

### B. Purification of Labelled ECR by Affinity Chromatography on a Fibrinogen Sepharose™ Column

### 1) Seguential Elutions of Fibrinogen Affinity Column

The labelled cell supernatant prepared above was precleared prior to purification by chromatography over a plain Sepharose™ CL4B column (Pharmacia) previously equilibrated with the TBS extraction buffer. For purification of the ECR by affinity chromatography, the flow-through containing the labelled ECR was collected from the plain Sepharose™ column and loaded onto a fibrinogen Sepharose™ column prewashed with 10 column volumes of TBS extraction buffer. The column was previously prepared by coupling 8 mg of purified fibrinogen prepared in Example 1A to one ml (approximately 0.333 grams of resin) of cyanogen bromide-activated (CNBr) Sepharose™ 4B according to manufacturer's instructions (Pharmacia LKB). The labelled ECR-containing solution was maintained on the fibrinogen column overnight at 4°C and was mixed occasionally to immobilize the ECR on the fibrinogen ligand. Following the maintenance period, the flow-through was collected and stored separately at 4°C.

The column was then washed with 10 column volumes of TBS extraction buffer. Prior to the EDTA elution of the ECR immobilized on the fibrinogen column, the column was first maintained with 300 *µ*l of a 1 mg/ml Arg-Gly-Glu (RGE) peptide solution dissolved in TBS extraction buffer to elute nonspecifically immobilized labelled proteins. The collection of the eluate was followed by a 10 minute waiting period before the next application of 300 *µ*l of the RGE solution to the column. The elution with RGE was repeated 10 times for a total of 10 collected fractions. For the second set of elutions to remove contaminating fibrinogen-bound labelled vitronectin receptor, a member of the integrin superfamily, the column was maintained with 300 *µ*l of a 1 mg/ml Arg-Gly-Asp (RGD) peptide solution dissolved in TBS extraction buffer. The RGD eluate was collected and the elution protocol was repeated 10 separate times as described for the RGE elution resulting in the collection of labelled vitronectin receptor over 10 fractions. Peak fractions were determined by gamma detection. The peptides used for the above-described elutions were synthesized using the classical solid-phase technique described by Merrifield, Adv. Enzymol., 32:221-296 (1969) as adapted for use with a model 430 automated peptide synthesizer (Applied Biosystems, Foster City, CA). Prepared polypeptide resins were cleaved by hydrogen fluoride, extracted and analyzed for purity by high-performance liquid chromatography (HPLC) using a reverse-phase C18 column manufactured by Waters Associates, Milford, MA.

Labelled ECR bound to the then vitronectin receptor-free fibrinogen column was then eluted with 10-20 mM EDTA dissolved in TBS extraction buffer. The 20 mM EDTA elution was preferred. The elution protocol was performed as described above resulting in the collection of purified ECR over 10 separate fractions. Peak fractions containing the eluted ¹²⁵I-labelled ECR were determined by gamma detection. The column was then washed with 10 column volumes of TBS extraction buffer followed by 3 column volumes of 1 M NaCl in TBS. The column was stored at 4°C after a final wash with at least 20 column volumes of PBS containing 0.02% sodium azide.

### 2) Characterization of the Purified Fibrinogen-Specific ECR The molecular weight of the fibrinogen

Sepharose™-purified ¹²⁵I-labelled ECR was determined by 7.5% SDS-PAGE with and without reduction with 3% betamercaptoethanol. Figure 1 shows the autoradiographic results of electrophoresis of aliquots of peak fractions from both the RGD and EDTA elutions of cell lysates prepared from cells either left untreated or treated with TNF as described in Example 2A. Lanes 1 and 3 show the RGD-eluted receptors isolated from cell lysates respectively prepared from untreated or TNF-treated cells. No bands are detectable in lane 1. However, in lane 3, two bands corresponding to the approximate molecular weights of 125 and 110 kD are present. These bands respectively correspond to alpha v and beta 3 subunits of the vitronectin receptor as described by Cheresh et al., Proc. Natl. Acad. Sci., 84:6471-6475 (1987), hereby incorporated by reference. Lanes 2 and 4, respectively EDTA-eluted ECR isolated from untreated and TNF-treated cells, reveal the presence of a lower molecular weight band of approximately 90-95 kD, the intensity of which is enhanced about 3-5 fold as a result of the induction of ECR expression by exposure to TNF. Thus, the EDTA-eluted fractions contained a non-RGD dependent ECR having a molecular weight of 90-95 kD distinct from the vitronectin receptor that binds to fibrinogen via the RGD tripeptide sequence (Cheresh et al., supra).

### C. Purification of Labelled ECR by Affinity Chromatography on a RGD Sepharose™ Column Followed by a Fibrinogen Sepharose™ Column

### 1) Sequential Column Chromatography

That the EDTA-eluted fibrinogen-binding ECR was a receptor distinct from vitronectin receptor was confirmed using a alternative approach of purifying ECR from labelled cell lysates by affinity chromatography over two different columns. The cell lysate prepared in Example 2A was first applied onto an RGD Sepharose™ column previously equilibrated with TBS extraction buffer to immobilize RGD-specific receptors to the Sepharose™-bound RGD. Coupling of RGD to CNBr-Sepharose™ was performed as described in Example 2A for preparation of a fibrinogen Sepharose™ column. After the overnight maintenance period for maximizing the interaction of the ¹²⁵I-labelled cell lysate containing RGD-dependent receptors with the Sepharose™-bound RGD, the flow-through was collected and applied on a fibrinogen Sepharose™ column as described in Example 2B. The RGD column was then washed as previously described. EDTA elution buffer was subsequently applied to the washed RGD column and fractions containing ¹²⁵I-labelled EDTA-eluted receptors were collected as described for the elution protocol in Example 2B. The characterization of the EDTA-eluted receptors from the RGD Sepharose™ column is described in Example 2C2) below.

The flow-through from the RGD column was maintained overnight with the fibrinogen column to allow for maximum interaction of the RGD-extracted cell lysate containing RGD-independent fibrinogen receptors with the Sepharose™-bound fibrinogen. After the maintenance period, the ¹²⁵I-labelled receptors bound to fibrinogen were eluted following the same procedure as described for the elution from the fibrinogen column in Example 2B. Ten fractions were collected from each of the sequential RGE, RGE and EDTA elutions. The characterization of the RGD- and EDTA-eluted material from the fibrinogen Sepharose™ column is described in Example 2C2) below.

### 2) Characterization of the RGD- Versus Fibrinogen-Dependent Receptors

Aliquots of the collected fractions from the EDTA elution of the RGD Sepharose™ column were electrophoresed adjacent to aliquots from both of the RGD and EDTA elutions from the fibrinogen Sepharose™ column under both nonreducing and reducing conditions to provide for an optimal comparison and characterization of the eluted receptors. Aliquots of the collected fractions were electrophoresed as described in Example 2B2). The results of autoradiographic exposure of the electrophoresed ¹²⁵I-labelled receptors are shown in Figure 2 in 8 lanes. Lanes 1 through 4 show migration of proteins under reducing conditions while lanes 5 through 8 show the migration of identical aliquots run under nonreducing conditions. The molecular weight determinations of the electrophoresed eluted receptors are made by comparison to ¹²⁵I-labelled molecular weight standards of 210, 107, 71 and 41 kD, respectively, myosin, beta-galactosidase, bovine serum albumin and ovalbumin. These markers are shown in lanes 4 and 8.

In lanes 3 and 7, the vitronectin receptor eluted with EDTA from the RGD-Sepharose™ column exhibits the characteristic profile of alpha v/beta 3 under reducing and nonreducing conditions. Unreduced alpha v has a molecular weight of 150 kD (lane 7, upper band) which is cleaved into two polypeptides of 125 and 25 kD under reducing conditions (lane 3, middle band - the 25 kD fragment has run off the gel). Unreduced beta 3 has a molecular weight of 90 kD (lane 7, lower band) which is increased to 110 kD under reducing conditions (lane 3, lower band).

In addition to the vitronectin integrin receptor having alpha v and beta 3 subunits, another integrin beta subunit, beta 1, was eluted from the RGD Sepharose™ column with EDTA. Alpha v has been shown to separately associate with both beta 3 and beta 1 as described by Vogel et al., J. Biol. Chem., 265:5934-5937 (1990). Beta 1 migrates as a 120 kD protein under nonreducing conditions (lane 7, middle band) which increases to 140 kD under reducing conditions (lane 3, upper band). Thus, the RGD-dependent vitronectin receptor consisting of alpha v/beta 3 subunits was eluted from both a fibrinogen Sepharose™ column with RGD and from a RGD column with EDTA.

In contrast, a different profile of the eluted proteins was obtained from fibrinogen affinity chromatography of cell lysates precleared on the RGD Sepharose™ column. As described above under Example 2Cl), the flow-through collected from the RGD Sepharose™ column lacking alpha v/beta 1 and beta 3 was then chromatographed on a fibrinogen Sepharose™. Since all the RGD-dependent receptors were removed by the first affinity column chromatography run, no ¹²⁵I-labelled elution products were obtained when the fibrinogen column was subjected to RGD elution (lanes 1 and 5, respectively, reduced and nonreduced conditions). However, with EDTA elution following the RGD elution, a single band of approximately 90-95 kD under nonreducing conditions (lane 6) was recovered. Under reducing conditions, the molecular weight of the EDTA-eluted fibrinogen receptor derived from human umbilical vein endothelial cells (HUVEC) referred to as ECR only slightly increased (lane 2). The determined molecular weights of the isolated ECR purified by either of the two approaches described in Examples 2B (sequential elutions of a single fibrinogen Sepharose™ affinity chromatography) or 2C (sequential affinity chromatography on separate affinity columns) were the same. Thus, the identical ECR was purified using two alternative approaches as shown in both Figures 1 and 2. In addition, the ECR was also purified by affinity chromatography on a fibrinogen Sepharosen™ column with EDTA without a prior elution step with RGD to remove other fibrinogen-binding receptors.

### D. Identification of the Purified Fibrinogen-Specific ECR as ICAM-1 by Immunoprecipitation

### 1) Immunoprecipitation of a 90-95 kD ECR with Anti-ICAM-1 Monoclonal Antibodies

Aliquots of fractions containing ¹²⁵I-labelled 90-95 kD ECR purified by either approach as described in Example 2B or 2C were used in immunoprecipitations to further identify the fibrinogen-binding ECR. Fifty to 100 *µ*l of peak fractions containing the ECR as determined by affinity chromatography as described above were separately admixed with 20 *µ* g of a mouse monoclonal anti-human Intercellular Adhesion Molecule-1 (ICAM-1) antibody commercially available from Becton Dickinson Immunocytochemistry Systems, Mountain View, CA. Separate aliquots were admixed with control antibodies. For the IgG control, a mouse monoclonal designated 1C10 commercially available from Telios, San Diego, CA, was used as a control which recognized a 130 kD endothelial cell surface protein. For the IgM control, an irrelevant IgM mouse monoclonal antibody was used. The admixtures were maintained on ice for one hour to form immune complexes. To immunoprecipitate or collect the formed immune complexes, 100 *µ*l of goat anti-mouse IgG coupled to agarose (Sigma Chemical Co.) at a ratio of 1:1.

Typically, 50 *µ*l of the eluted ECR in a peak fraction was admixed with 50 *µ*l of the goat anti-mouse IgG-coupled agarose and maintained on ice for 30 minutes. The immune complexes bound to goat anti-mouse agarose were subsequently pelleted by centrifugation at 10,000 X g for one minute at 4°C. The resultant supernatants were removed by aspiration and the pellets were resuspended in TBS extraction buffer. The pellets were washed 3 times and finally resuspended in Laemmli sample buffer for SDS-PAGE analysis against the molecular weight standards described above. Following electrophoresis, the gel was dried and autoradiographed. A single 90-95 kD band was evident on the developed films indicating that the 90-95 kD fibrinogen affinity purified ECR was in fact ICAM-1 as determined by immunoprecipitation with a mouse monoclonal antibody raised against human ICAM-1.

### 2) Immunoprecipitation of a 90-95 kD ECR with Anti-HUVEC Monoclonal Antibodies

Immunoprecipitations as described above were also performed with mouse monoclonal antibodies raised against intact unstimulated HUVEC. The preparation and characterization of four such monoclonal antibodies is described in Example 4. Fifty *µ*l of the IgM monoclonal antibody designated 2E12 was admixed with 50 *µ*l of the same fraction used in the immunoprecipitations with the commercially available anti-ICAM-1 antibody. After electrophoresis and exposure of the autoradiographic film, a 90-95 kD band was evident. Thus, the monoclonal antibodies directed against HUVEC cell surface proteins immunoprecipitated the same 90-95 kD protein as that immunoprecipitated with a commercially available mouse monoclonal antibody to human ICAM-1. The ECR that binds to fibrinogen via a RGD-independent binding site is now identified as ICAM-1 as determined by affinity chromatography analysis (Examples 2B and 2C). The binding of the ECR, hereinafter referred to as ICAM-1, to an RGD-independent binding site in fibrinogen is a novel finding.

### 3. Confirmation of an RGD-Independent Fibrinogen Receptor on Endothelial Cells (HUVEC)

Adhesion of leukocytes to vascular endothelium is one of the earliest events in a variety of immune-inflammatory reactions. At the molecular level, leukocyte adhesion to endothelial cells is a redundant mechanism, supported by the regulated recognition of a disparate set of membrane receptors expressed on both leukocytes and endothelial cells, the latter of which may either be in a resting or a cytokine-stimulated state. A novel set of molecular interactions participating in leukocyte adhesion are now identified. Fibrinogen has been shown to interact leukocytes (monocytes, peripheral mononuclear cells and various cell lines) via the integrin CD11b/CD18, also referred to as Mac-1, as described by Altieri et al., J. Biol. Chem., 265:12119-12122 (1990), hereby incorporated by reference. Studies of the interaction of fibrinogen with endothelial cells in vitro has now resulted in the discovery of an endothelial cell surface membrane receptor that binds an RGD-independent site on fibrinogen. Presented herein are data showing that the interaction between circulating leukocytes and endothelial cells is mediated by a bridging effect of different parts of the fibrinogen molecule to distinct cell surface membrane receptors expressed on each cell type.

### A. Demonstration of Fibrinogen Binding to HUVEC

### 1) Preparation of Iodinated Fibrinogen

Fibrinogen was iodinated using the Iodogen™ method. Briefly, Iodogen™ was dissolved in dichloromethane for a final concentration of 1 *µ*g/ml and 170 *µ*l of dissolved Iodogen™ that was dried in the bottom of a glass tube. Fibrinogen, prepared in Example 1A, was resuspended in 0.055 M sodium citrate buffer, pH 7.4, for a final concentration of 5 *µ*g/ml. Two hundred *µ*l of dissolved fibrinogen solution was placed into the Iodogen™-coated tube with 700 *µ*Ci of carrier-free sodium iodide. The admixture was maintained on ice for 20 minutes with occasional agitation. To stop the iodination reaction, the admixture was removed from the tube and gel filtered on a Sepharose™ G-25 coarse column (100 x 2.5). Fractions of iodinated fibrinogen were determined by trichloroacetic acid precipitable counts. The labelled fibrinogen produced was radiolabelled to a specific activity of 0.3 *µ* Ci/*µ*g of protein. Labelled fibrinogen was used in the binding and inhibition of binding assays described herein at a concentration of 50 *µ*g/ml whereas unlabelled fibrinogen was generally used at a concentration of 500 *µ*g/ml.

### 2) Analysis of Dose Dependency

To determine if fibrinogen bound to a cell surface HUVEC receptor and if so, at what concentrations, increasing concentrations from 0.01 micromolar (*µ*M) up to 0.44 *µ*M ( 0.14 *µ*M is equivalent to 50 *µ*g/ml; 0.29 *µ*M is equivalent to 100 *µ*g/ml and 0.44 *µ*M is equivalent to 150 *µ*g/ml) of iodinated fibrinogen (¹²⁵I-Fg) prepared above were separately admixed to monolayers of HUVEC cells that were previously washed two times with serum-free RPMI 1640. The HUVEC cell cultures were initially plated in individual wells of a 48 well plate coated for tissue culture (Costar Corp., Cambridge, MA) as described in Example 2A for culturing of cells in T75 flasks. The divalent cation, presented as calcium chloride (CaCl₂), at a concentration of 2.5 mM was also admixed into the cell-fibrinogen admixtures. The inhibitor of fibrin polymerization, PPack (D-phenyl-1-prolyl-1 arginine chloramethyl; Calbiochem Boehring), was admixed to the cell admixtures at a concentration of 100 mM. PPack was present in all assays where it was necessary to prevent the polymerization of fibrinogen into fibrin.

The resultant admixtures were maintained at 22°C for 45 minutes to allow for fibrinogen to bind to the plated HUVEC. After the maintenance period, the cells were washed two times with serum-free RPMI 1640 to remove unbound fibrinogen. The cells were then solubilized in 10% SDS and the radioactivity associated under the maintenance conditions was quantitated in a gamma counter.

The resultant data is plotted in Figure 3 as ¹²⁵I-labelled fibrinogen bound in counts per minute (cpm) per well (X 10⁻³) on the Y-axis against increasing concentrations of ¹²⁵I-labelled fibrinogen (X 10⁻⁷ M) on the X-axis. The data shows that ¹²⁵I-labelled fibrinogen binds saturably at a concentration of approximately 0.36 *µ*M to monolayers of unstimulated HUVEC.

### 3) Analysis of Effect of HUVEC Stimulation by Exposure to TNF or Lipopolysaccharide on Binding of Fibrinogen

To determine the effect that known stimulators of HUVEC have on the binding characteristics of fibrinogen to HUVEC, dose-response experiments were performed as described in Example 3A2) on untreated HUVEC and TNF or lipopolysaccharide (LPS, Genzyme))-stimulated HUVEC. TNF and LPS were separately admixed at the respective concentrations of 5 nanograms (ng)/ml and 1.0 *µ*g/ml to monolayers of HUVEC and maintained at 37°C for 4 hours prior to the admixture of the labelled fibrinogen ranging in concentration from 0.01 *µ*M up to 0.36 *µ*M.

The resultant data is plotted in Figure 4 as ¹²⁵I-labelled fibrinogen bound in molecules per cell (X 10⁻⁶) on the Y-axis against increasing concentrations of ¹²⁵I-labelled fibrinogen (X 10⁻⁷ M) on the X-axis. Under stimulation with either TNF or LPS, the number of labelled fibrinogen molecules bound per cell doubled in comparison to those bound to unstimulated cells. Thus, the increase of fibrinogen binding to ICAM-1 receptor on HUVEC is cytokine or immunostimulant mediated.

### 4) Analysis of Binding of D₃₀ to HUVEC

Binding assays described above in Example 3A2) were also performed with the fibrinogen homolog, D₃₀, to determine if that region of fibrinogen also immunoreacted with HUVEC. Since D₃₀ was known to bind to leukocytes via the Mac-1 receptor as described by Altieri et al., J. Biol. Chem., 265:12119-12122 (1990), these experiments were performed to determine if region of fibrinogen mediating the binding of leukocytes to HUVEC was contained within the D₃₀ fragment. For this analysis, the D₃₀ fragment of fibrinogen prepared in Example 1B was labelled with ¹²⁵I as described for labelling of fibrinogen above. Iodinated D₃₀ was admixed to HUVEC monolayers at a concentration of 10 *µ*g/ml to form a binding complex. After washing the cells to remove the unbound D₃₀ as described in Example 3A2) above, the cells were solubilized and the amount of bound radioactivity was determined. The binding of D₃₀ to HUVEC was maximal at 120 minutes of the maintenance period with approximately 60,000 cpm. The binding of D₃₀ was specifically competed by admixture of 50 fold molar excess of cold fibrinogen thus confirming that D₃₀ specifically bound to a fibrinogen binding site on HUVEC. Myoglobin, a nonspecific protein, did not inhibit the binding of D₃₀ to HUVEC.

Confirmation of the specificity of D₃₀ binding to HUVEC was obtained by inhibiting the binding of D₃₀ to ICAM-1 transfected cells prepared in Example 4 in the presence of the 14E11 IgG monoclonal antibody also prepared in Example 4. The inhibition of binding assays were performed as described in Example 5. The anti-ICAM-1 BD monoclonal antibody described in Example 4 was also used in the assay. Both 14E11 and the anti-ICAM-1 BD monoclonal antibodies, at a concentration of 20 *µ*g/ml in the presence of MnCl₂, specifically inhibited the binding of D₃₀ to HUVEC. Approximately 3000 and 7500 cpm were recovered from the binding of D₃₀ in the presence of CaCl₂ and MnCl₂, respectively, in the absence of any inhibitors. With 14E11 and MnCl₂, D₃₀ binding to the transfectants was completely inhibited. The portion of fibrinogen containing D₃₀, therefore, binds to the fibrinogen receptor on HUVEC and to surface expressed ICAM-1 on transfectants.

The fibrinogen bridge, however, is not contained within the D₃₀ fragment as determined by inhibition of binding assays in the presence of peptides derived from the Mac-1 receptor binding site on D₃₀. The D₃₀-derived peptides did not block the binding of either fibrinogen or D₃₀ to the HUVEC. Therefore, the bridging site of fibrinogen that binds to the endothelial fibrinogen receptor is within the D₃₀ fragment but is not the same region that mediates the binding of D₃₀ or fibrinogen to Mac-1 on leukocytes.

### B. Demonstration of Fibrinogen Bridging the Binding of Mac-1-Bearing Cells to an RGD-Independent Fibrinogen Receptor on HUVEC

### 1) Analysis of Dose Dependency Over Time

In vivo, circulating leukocytes have been shown to bind to the apical surface of endothelial cells. In addition, experiments have been performed in vitro where the monocytic cultured cell line, THP-1 having the ATCC accession number TIB 202, (ATCC, Bethesda, MD), was shown to bind directly to unperturbed HUVEC in the presence of divalent cations with or without stimulation with 1 *µ*M of the chemotactic peptide, N-formyl-methionyl-leucyl-phenylalanine (N-FMLP) (Sigma Chemical Co.) as described by Altieri, J. Immunol., 147:1891-1898 (1991), hereby incorporated by reference. To determine whether this event was the result of a fibrinogen mediated bridging phenomenon, in vitro cell attachment binding assays were performed.

For these assays, HUVEC were plated in the medium described in Example 2A at a density of approximately 1-5 X 10⁴ cells/well into flat-bottom microtiter wells of a 96 well tissue-culture treated plate. The cells were then washed in serum-free RPMI 1640 and further maintained with ⁵¹-Chromium-labelled (⁵¹Cr) THP-1 cell suspensions previously exposed to different concentrations of unlabelled fibrinogen or left untreated. To label THP-1 cells, serum-free suspensions of the cells at a concentration of 1 X 10⁷ cells/ml were labelled with 0.5 mCi ⁵¹Cr (Na₂CrO₄ having a specific activity of 487.4 mCi/mg, NEN Du Pont de Nemours) for 2 hours at 37°C with incorporation of an average of 12 to 20 cpm/THP-1 cell. The labelled cells were then washed twice at room temperature with serum-free RPMI 1640 and resuspended in the same medium at a concentration of 5 X 10⁵ cells/ml. The labelled cells were used in the assays within 2 hours from the labelling procedure. For the assays, the cells were pre-stimulated with 1 *µ*M N-FMLP in the presence of 1 mM CaCl₂ and 100 mM PPack. The resultant N-FMLP-stimulated THP-1 cell suspensions were then separately admixed with the following: 1) Medium without any admixed fibrinogen as a control; 2) Fibrinogen at 1.2 mg/ml and at 2.5 mg/ml concentrations; and 3) Normal human plasma (NHP) diluted 1:2 and 1:50. Purified fibrinogen was prepared as described in Example 1. Fibrinogen was present in undiluted normal human plasma at the concentration of approximately 1-3 mg/ml. The resultant admixtures were maintained for 20 minutes at 22°C to allow for the binding of fibrinogen, either purified or present in NHP, to the Mac-1 receptor on the surface of the THP-1 cells.

The resultant fibrinogen-bound THP-1 cells were then admixed to the washed immobilized HUVEC described above to allow for the binding of a non-Mac-1 receptor binding site on fibrinogen to the RGD-independent fibrinogen receptor on the surface of HUVEC, thereby resulting in the binding of THP-1 cells to HUVEC via a fibrinogen bridge. The admixtures were maintained at 37°C to allow for adhesion. At selected time intervals between 1 to 60 minutes, the HUVEC monolayers were gently washed five times with serum-free RPMI 1640 to remove nonadherent or loosely adherent THP-1 cells to which fibrinogen was initially immobilized. The adherent cells were then solubilized in 10% SDS and the cell lysate was quantitated in a beta scintillation counter. Spontaneous ⁵¹Cr release from THP-1 cells was always less than 2% during the adhesion assay. The number of specifically attached THP-1 cells was determined by dividing the cpm harvested by the cpm/cell.

The results of these experiments are shown in Figure 5A and Figure 5B where the data is expressed as numbers of ⁵¹Cr-labelled THP-1 cells (X 10⁻³) on the Y-axis plotted against the assay time on the X-axis. THP-1 cells did not significantly bind to immobilized HUVEC in the absence of fibrinogen throughout the time course. In contrast, THP-1 cells maintained in the presence of 1.2 mg/ml fibrinogen exhibited significant increases of binding to HUVEC over the time course with the maximum cell attachment occurring at 10,000 cells (Figure 5A). In the presence of 2.5 mg/ml of fibrinogen, TPC-1 cell attachment had not saturated at the end of 60 minutes where approximately 18,000 THP-1 cells were attached to fibrinogen (Figure 5A). Similar binding curves were obtained in the presence of NHP shown in Figure 5B. Maximum THP-1 cell attachment of approximately 23,000 cells was obtained after 40 minutes in the presence of NHP diluted 1:2 which contains approximately 0.5-1.5 mg/ml fibrinogen. NHP diluted 1:50 exhibited a profile comparable to that seen with 1.2 mg/ml of purified fibrinogen. Thus, the time-dependent binding of THP-1 cells to HUVEC was fibrinogen-dependent confirming that fibrinogen, either purified or present in NHP, serves as a protein bridge between the two cell types.

### 2) Analysis of Temperature Dependency

To determine the effects that temperature has on the ability of fibrinogen to mediate the binding of ⁵¹Cr-labelled THP-1 cells to monolayers of HUVEC, cell adhesion assays as described in Example 3B1) were performed at 22°C and at 37°C over the course of one hour. The assays were performed in an identical manner as described above with the exception that 500 *µ*g/ml of fibrinogen was used instead of 1.2 or 2.5 *µ*g/ml. The maximum binding of THP-1 cells to HUVEC at 22°C was 7500 cells after 40 minutes. At 60 minutes, the binding decreased to 5000 cells. The data is significant in comparison to the approximately 2000 THP-1 cells attached in the absence of fibrinogen. However, at 37°C, approximately 20,000 THP-1 cells attached to HUVEC after 40 minutes against a background binding of approximately 5000 cells in the absence of fibrinogen. The binding of ⁵¹Cr-labelled THP-1 cells to HUVEC, thus, is maximized at the physiologic temperature of 37°C with physiologic concentrations of fibrinogen.

### 3) Analysis of Cell Type Specificity

Cell adhesion binding assays described above were performed on bovine aortic endothelial cells (BAE) to determine if fibrinogen could mediate the binding of ⁵¹Cr-labelled THP-1 cells to endothelial cells from a different source. Cell cultures were prepared from bovine aorta following procedures known to one skilled in the art. THP-1 cells were either left untreated or treated with 500 *µ*g/ml fibrinogen over a 60 minute time course. At selected time points, the cells were harvested as described in Example 3B1) and the number of attached THP-1 cells were determined as previously described. In the absence of fibrinogen, THP-1 cells did not significantly bind to BAE cells (less than 5000 cells attached) after an initial rise of attachment peaking at 20 minutes. However, in the presence of fibrinogen, approximately 20,000 cells bound to BAE cells after a 40 minute maintenance period. This maximum binding dropped off at 60 minutes to approximately 15,000 attached cells. Binding of THP-1 cells to HUVEC was done in parallel as a control for the experiment; the non-saturable binding of THP-1 cells in the presence of fibrinogen was maximal at 60 minutes with approximately 25,000 cells attached. Thus, fibrinogen mediates the binding of THP-1 cells not only to human endothelial cells but also to bovine-derived endothelial cells.

### 4. Preparation of Anti-Endothelial Cell Monoclonal Antibodies to an RGD-Independent Fibrinogen Receptor on HUVEC

### A. Preparation of Immunogen

HUVEC, cultured as described in Example 2A but without TNF or LPS stimulation, were prepared for use as immunogens in order to raise monoclonal antibodies against HUVEC surface proteins for eventual screening by assaying the inhibition of ¹²⁵I-labelled fibrinogen binding to HUVEC cultures. For the immunizations, 10 X 10⁶ cells harvested from the culture plates by treatment with 4.0 mM EDTA and resuspended in saline were injected into mice as described below.

### B. Preparation of Monoclonal Antibodies to an RGD-Independent Fibrinogen Receptor on HUVEC

The HUVEC, prepared as immunogens according to Example 4A, were injected intraperitoneally (i.p.) into separate Balb/c ByJ mice (The Scripps Research Institute Vivarium, La Jolla, CA). The mice received booster injections at 1, 3 and 5 weeks. The last boost was 4 days prior to fusion.

The animals so treated were sacrificed and the spleen of each mouse was harvested. A spleen cell suspension was then prepared. Spleen cells were then extracted from the spleen cell suspension by centrifugation for about 10 minutes at 1000 rpm, at room temperature. Following removal of the resultant supernatant, the cell pellet was resuspended in 5 ml cold ammonium chloride (NH₄Cl) lysing buffer, and was maintained for about 10 minutes.

Ten ml of Dulbecco's Modified Eagle Medium (DMEM) (Whittaker M.A. Bioproducts) and Hepes buffer were admixed to the lysed cell suspension to form an admixture, and that admixture was centrifuged for about 10 minutes at 1000 rpm at room temperature.

After the resultant supernatant was decanted, the pellet was resuspended in 15 ml of DMEM and Hepes and was centrifuged for about 10 minutes at 1000 rpm at room temperature. The above procedure was repeated.

The pellet was then resuspended in 5 ml DMEM and Hepes. An aliquot of the spleen cell suspension was then removed for counting. Fusions were accomplished in the following manner using the non-secreting mouse myeloma cell line P3X63Ag8.653.1, a subclone of line P3X63Ag8.653 (ATCC Accession Number CRL 1580). With a myeloma to spleen cell ratio of about 1 to 10 or about 1 to 5, a sufficient quantity of myeloma cells were centrifuged into a pellet, washed twice in 15 ml DMEM and Hepes, and then centrifuged for 10 minutes at 1000 rpm at room temperature.

Spleen cells and myeloma cells were combined in round bottom 15 ml tubes. The cell mixture was centrifuged for 10 minutes at 1000 rpm. at room temperature and the supernatant was removed by aspiration. Thereafter, 200 *µ*l of 50 percent (weight per volume) aqueous polyethylene glycol 4000 molecular weight (PEG) at about 37°C were admixed with the pellet using a 1 ml pipette with vigorous stirring to disrupt the pellet. The cells were then gently mixed for between 15 and 30 seconds. The resultant cell mixture was centrifuged 4 minutes at 700 rpm.

At about 8 minutes from the time of adding the PEG, 5 ml of DMEM plus Hepes buffer were admixed slowly to the pellet, without disturbing the cells. After 1 minute, the resulting admixture was broken up with a 1 ml pipette and was maintained for an additional 4 minutes. This admixture was centrifuged for 7 minutes at 1000 rpm. The resultant supernatant was decanted, 5 ml of HT (hypoxanthine/thymidine) medium were slowly admixed to the pellet and the admixture was maintained undisturbed for 5 minutes. The pellet was then broken into large chunks and the final cell suspension was placed into T75 flasks (2.5 ml per flask) into which 7.5 ml HT medium had been previously placed. The resulting cell suspension was maintained at 37°C to grow the fused cells. After 24 hours, 10 ml of HT medium were admixed to the flasks followed 6 hours later by admixture of 0.3 ml of 0.04 mM aminopterin. Forty-eight hours after the fusion, 10 ml of HAT (hypoxanthine/aminopterin/thymidine) medium were admixed to the flasks.

Three days after fusion, viable cells were plated out in 96-well tissue culture plates at about 2 x 10⁴ viable cells per well (768 total wells) in HAT buffer medium as described in Kennett et al., Curr. Top. Microbiol. Immunol., 81:77 (1978). The cells were fed seven days after fusion with HAT medium and at approximately 4-5 day intervals thereafter as needed with HT medium. Growth was followed microscopically and culture supernatants were collected about two weeks later.

### C. Immunoscreening of Monoclonal Antibodies by Cell Adhesion Assays

The culture supernatants from HAT resistant cultures prepared above were subsequently assayed for the presence of HUVEC RGD-independent fibrinogen receptor antibodies by the binding assays and cell adhesion assays respectively described in Example 3A and 3B, and further described in Example 5. Culture supernatants were tested for their ability to inhibit the binding of ¹²⁵I-labelled fibrinogen to monolayers of HUVEC. For this assay, the monolayers were maintained in the presence of hybridoma culture supernatant and 2.5 mM CaCl₂ for 30 minutes at 37°C. After the maintenance period, the supernatant-treated HUVEC were washed once with RPMI 1640. Labelled fibrinogen was then admixed to the treated HUVEC at a concentration of 50 *µ*g/ml in the presence of 2.5 mM CaCl₂ and 100 mM PPack. The resultant admixtures were maintained for 30-60 minutes at 22°C. The fibrinogen-treated HUVEC were then washed, solubilized and counted as described in Example 3A.

Supernatants were also screened for their ability to block the binding of ⁵¹Cr-labelled THP-1 cells previously exposed to fibrinogen to HUVEC cells. The assay was performed essentially as described for the cell adhesion assay in Example 3B with the exception that the HUVEC were separately maintained with hybridoma supernatants for 30 minutes at 37°C as described above. Following the antibody exposure, the cells were washed once with culture medium prior to the admixture of the fibrinogen-bound and labelled THP-1 cells at the desired concentrations as described in Example 3B.

Hybridoma culture supernatants that produced an antibody of this invention which effectively blocked the fibrinogen-mediated binding of THP-1 cells to monolayers of HUVEC were then selected for subsequent purification and characterization. Hybridoma cultures producing antibodies against RGD-independent fibrinogen receptors (also referred to as a fibrinogen binding site) on HUVEC were identified. Four separate antibodies, designated 14E11, 16G8, 2E12 and 2B12, were obtained. 14E11 was determined to be an IgG while the remaining monoclonals were determined to be IgMs. The monoclonal antibodies, specific for an endothelial cell RGD-independent fibrinogen receptor (also referred to as anti-ECR equivalent to anti-ICAM-1 based on the affinity chromatography analysis in Example 2), were shown to immunoreact with HUVEC in addition to the purified ECR eluted from a fibrinogen Sepharose™ column with EDTA as described in Example 2D, and to not immunoreact with VNR.

### D. Purification of the Selected Monoclonal Antibodies

The four hybridomas secreting anti-ECR antibodies as described in Example 4C were injected into 10-week old Balb/c mice as described below to produce ascites fluid.

To that end, separate sets of 10-week old Balb/c mice were primed with 0.3 ml of mineral oil and then injected intraperitoneally with 5 x 10⁶ hybridoma cells for each monoclonal. The average time for development of ascites was 9 days. Following clarification by centrifugation at 15,000 x g for 15 minutes at room temperature, ascites fluids produced by hybridomas were pooled and stored frozen at -20°C to form monoclonal antibody compositions.

The ascites-produced monoclonal antibodies were further purified by fast protein liquid chromatography (FPLC) using a Pharmacia Mono Q HR5/5 anion exchange column (Pharmacia) using a 0-0.5 M NaCl gradient in 10 mM Tris-HCl at pH 8.0 following directions supplied with the column. The FPLC-treated monoclonal antibodies were then concentrated using an Amicon stirred ultrafiltration cell (Danvers, MA; PM 30 membrane) to a concentration of 1 mg/ml, dialyzed into PBS and stored at -70°C to form purified MAb.

The monoclonal antibody, 14E11, was further affinity purified using a affinity purification kit, Affi-Prep, according to the manufacturer's instructions (Bio-Rad, Richmond, CA). The IgM monoclonal antibodies were further purified by hydroxylapatite gel filtration over a Bio-Gel HPHT hydroxylapatite column according to manufacturer's instructions (Bio-Rad). These purified antibodies were used in subsequent binding assays, Western immunoblots and immunoprecipitations are described in the Examples.

### E. Confirmation of the Immunospecificity of the Monoclonal Antibodies to an RGD-Independent Fibrinogen Receptor on HUVEC

The immunospecificity of the monoclonal antibodies, 14E11, 16G8, 2E12 and 2B12, was confirmed by a number of approaches. Firstly, as described in Example 2D, immunoprecipitation of the fibrinogen affinity chromatography-purified ECR was performed using 2E12 in comparison to immunoprecipitation with a commercially available anti-ICAM-1 antibody (Becton Dickinson, referred to as BD). The 90-95 kD purified ECR was immunoprecipitated with both of the antibodies indicating that the 2E12 antibody had the same immunospecificity as the anti-ICAM-1 antibody and that the purified ECR was ICAM-1. The exact epitopes of the ECR (ICAM-1) recognized by the antibodies, 2E12 and Becton Dickinson anti-ICAM-1, have not been determined. Based on the blotting profile obtained by Western immunoblot analysis described below and by the inhibition of binding data presented in Example 5, the epitopes are likely to be unique.

Fluorescent Activated Cellscan (FACscan) analysis was performed on an fibroblast-like cell line which was genetically engineered to express the recombinant form of ICAM-1 on the surface of the cells as described by Seed et al., Nature, 331:624-627 (1988), hereby incorporated by reference. Briefly, a cDNA library, constructed using RNA prepared from HL-60 cells induced with 12-O-tetradecanoyl phorbol 13-acetate (TPA), was transfected into COS cells. The cells expressing surface antigens were screened by panning with anti-ICAM monoclonal antibodies, designated 8F5 and 84H10, resulting in the selection of a cDNA clone in a transfected cell expressing ICAM-1. These transfectants were used in FACscan analysis to confirm the immunospecificity of the anti-ECR antibodies of this invention.

For the analysis, 1 X 10⁶ ICAM-1 transfectant cells in suspension were separately admixed with 1 *µ*g/ml affinity-purified 14E11, an admixture of hydroxylapapatite purified of all three IgM monoclonal antibodies (16G8, 2E12 and 2B12), with the anti-ICAM-1 BD monoclonal antibody and a control monoclonal antibody designated PMI-I having the ATCC Accession Number HB 9476. The latter recognizes the C-terminal hGPIIb fragment of the receptor GPIIb/IIIa found on platelets. The separate admixtures were maintained for 30 minutes at 4°C to form immunoreaction products. After 3 washes in serum-free RPMI 1640, the immunoreacted transfected cells were admixed with fluorescein-conjugated goat anti-mouse immunoglobulins and maintained for 30 minutes at 4°C to form secondary immunoreaction products. After washing 3 times, the cells were subjected to flow cytometry on a Becton Dickinson IV/40 fluorescence activated cell sorter.

The results of the FACscan revealed that 16G8, 2E12 and 2B12 monoclonal antibodies specifically immunoreacted with the ICAM-1-expressing transfectants comparably to that seen with the anti-ICAM-1 BD antibody. 14E11, however, did not specifically immunoreact with the ICAM-1-expressing transfectants as its profile overlapped that seen with the control PMI-I antibody. Since the ICAM-1 expressed on the transfectants is known to be unglycosylated, the lack of immunoreactivity of 14E11 with the cells is mostly likely due to this reason. In the initial screen of the hybridomas, the 14E11 monoclonal antibody did block the binding of ¹²⁵I-labelled fibrinogen to HUVEC as well as block the binding of THP-1 cells via a fibrinogen bridge to HUVEC cells.

That the 14E11 monoclonal antibody specifically recognized the ECR identified as ICAM-1 was confirmed by Western immunoblot. Both HUVEC and Daudi cells were used for the blotting. Daudi is a Burkitt's lymphoma human cell line that expresses high levels of ICAM-1 and is available from ATCC having the ATCC Accession Number CCL 213. Cell lysates were prepared from cultures of each cell type as described for preparation of a cell lysate in Example 2A with the exception that the cells were lysed with 0.5% Triton-X 100 and 0.5% NP-40. After centrifugation as described in Example 2A, aliquots of each resultant supernatant were electrophoresed into multiple lanes by 10% SDS-PAGE.

Following the electrophoresis, the proteins in the gel were transferred electrophoretically to nitrocellulose for subsequent immunoreactions. After the nitrocellulose blot was maintained for 2 hours at room temperature immersed in a solution of non-fat dry milk (Blotto) to block nonspecific binding sites, it was then cut into strips to isolate each individual lane of electrophoresed proteins, 5 for Daudi and 8 for HUVEC. The nitrocellulose strips were then separately immunoreacted with two control antibodies, 2E1 and PMI-I, purified 14E11 IgG, 14E11 culture supernatant and anti-ICAM-1 BD monoclonal antibody for 1 hour at room temperature to form primary immunoreaction products. The concentration of the primary antibodies added was approximately 10 *µ*g/ml. The immunoreacted blots were then washed 4 times with PBS for 5 minutes each. The washed blots were then immersed for 1 hour at room temperature in a solution of secondary ¹²⁵I-labelled goat anti-mouse antibodies (Zymed Laboratories Inc., San Francisco, CA) to form secondary immunoreaction products. The immunoreacted blots were then washed 4 times with PBS and exposed to X-ray film for the detection of immunoreacted electrophoresed proteins from the cell lysate supernatants. Additional controls included reacting the strips with a only the labelled secondary antibody.

The results of the Western blot are shown in Figure 6. The relative molecular weights of the electrophoresed proteins in the cell lysate supernatants were determined by comparison with a set of radiolabelled molecular weight markers of 97, 66, 45, 30 and 21 kD shown in lane left of the first set of 5 Daudi lanes and left of the second set of 8 HUVEC lanes. Lanes designated 1-5 at the bottom of the blot for both Daudi and HUVEC were respectively immunoreacted with 2E1, PMI-I, affinity purified 14E11, 14E11 culture supernatants and the anti-ICAM-1 BD monoclonal antibodies. A 90-95 kD band was detected in both Daudi and HUVEC cell lysate supernatants with the affinity purified 14E11 monoclonal antibody shown in number 3 labelled lanes. In addition, the affinity purified 14E11 immunoreacted with a protein of approximately 50-55kD in lane 3 of the HUVEC-electrophoresed proteins. While the anti-ICAM-1 BD antibody slightly immunoreacted with the Daudi proteins, it strongly immunoreacted with only the 90-95 kD band in the HUVEC electrophoresed proteins. The apparent difference in blotting patterns between the 14E11 and anti-ICAM-1BD monoclonal antibodies supports the position that they recognize separate epitopes on the ICAM-1 molecule. The control primary antibodies, 2E1 and PMI-I, immunoreacted with the electrophoresed Daudi and HUVEC proteins as predicted based on prior characterizations of binding specificities. Thus, the 14E11 IgG monoclonal antibody specifically recognized the 90-95 kD cell surface protein isolated from both Daudi and HUVEC comparable to that seen with the anti-ICAM-1 BD antibody by both Western blot described herein and by immunoprecipitation described in Example 2D.

In addition, as shown in Example 5, the affinity purified 14E11 antibody was completely effective at inhibiting the binding of THP-1 cells via the fibrinogen bridge to HUVEC.

### 5. Inhibition of Fibrinogen-Mediated Binding of Leukocytes to Endothelial Cells

### A. Inhibition of Fibrinogen-Mediated Binding of THP-1 Cells to HUVEC Using Fibrinogen Analogs

### 1) Excess Cold Fibrinogen

Binding of ¹²⁵I-labelled fibrinogen binding to HUVEC assays were performed in the presence of 50 molar excess of unlabelled fibrinogen in order to confirm the specificity of binding as shown in Example 3A. The binding assay was performed as described in Example 3A with the exception that unlabelled fibrinogen was admixed to HUVEC at a 50 molar excess (approximately 7.5 X 10⁻³ M fibrinogen) concurrently with 50 *µ*g/ml of ¹²⁵I-labelled fibrinogen. The protein-cell admixtures were maintained at 22°C for 30 minutes to allow for binding of fibrinogen to HUVEC. In addition to cold fibrinogen, a 50 fold molar excess of BSA was separately admixed as a control. The amount of fibrinogen bound under the above conditions was quantitated as described in Example 3A. The uninhibited total binding of ¹²⁵I-labelled fibrinogen saturated after 30 minutes at approximately 4000 cpm per well. The BSA did not inhibit the binding and thus ¹²⁵I-labelled fibrinogen exhibited a similar profile in the presence of BSA. Unlabelled fibrinogen, however, competed the binding of the labelled fibrinogen to half that of the total uninhibited binding. The binding of fibrinogen to the HUVEC surface fibrinogen receptor is therefore specific.

The specificity of binding to an RGD-independent fibrinogen receptor was confirmed in competition assays performed as described above in the presence of monoclonal antibodies against the beta subunit of the RGD-dependent VNR and in the presence of RGD-containing peptides. For the assays in which the ability of anti-VNR monoclonal antibodies, designated mAb 609 and mAb 7E3, to inhibit the binding of ¹²⁵I-labelled fibrinogen to HUVEC, the antibodies were separately maintained at a concentration of 25 *µ*g/ml with HUVEC for 20 minutes at 37°C. After the maintenance period, the antibody-treated HUVEC were washed once with serum-free RPMI 1640 to remove any unbound antibody. Labelled fibrinogen was then admixed to the treated HUVEC at a concentration of 50 *µ*g/ml in the presence of 2.5 mM CaCl₂ and 100 mM PPack. The resultant admixtures were maintained for 10-30 minutes at 22°C. The fibrinogen-treated HUVEC were then washed, solubilized and counted as described in Example 3A.

The results are shown in the bar graphs in Figure 7 where the amount of ¹²⁵I-labelled fibrinogen bound to HUVEC in cpm/well (X 10⁻³) is plotted on the Y-axis against the length of time labelled fibrinogen was maintained with HUVEC. Each part of the bar graph is separately identified for each time point as follows: total binding (no inhibitors admixed), +Fg (fibrinogen) admixed, +mAb 609; and +mAb 7E3. Cold fibrinogen that was added in 50 molar excess as described above almost completely inhibited the binding of ¹²⁵I-labelled fibrinogen to HUVEC. The VNR antibodies, however, had no inhibitory effect as anticipated by the results with the affinity chromatography in Example 2. Thus, fibrinogen binds to an receptor on HUVEC that is not VNR.

To confirm that the fibrinogen binding site on HUVEC was not RGD dependent, inhibition assays performed as described above for inhibiting with antibodies except that they were done in the presence of 1.0 mM of an RGD-containing peptide. A control RGE-containing peptide was also included in the assay. At selected time points over the time course of one hour, the HUVEC were harvested as described in Example 3A.

The resultant data are shown in Figure 8 where the amount of ¹²⁵I-labelled fibrinogen bound to HUVEC in cpm/well (X 10⁻³) is plotted on the Y-axis against the length of time labelled fibrinogen was maintained with HUVEC. Neither the RGD- nor the RGE-containing peptide had any inhibitory effect of the binding of labelled fibrinogen to HUVEC as compared to the total binding of the labelled fibrinogen in the absence of any inhibitor. The RGD- and the RGE-containing peptide data are shown respectively as the lines indicated by the solid and open squares. Cold fibrinogen completely inhibited the binding of labelled fibrinogen as expected.

In summary, an excess of unlabelled fibrinogen completely inhibits the binding of labelled fibrinogen to HUVEC and this binding is mediated through an RGD-independent fibrinogen receptor that is not VNR.

### 2) Anti-ECR (ICAM-1) Monoclonal Antibodies

The monoclonal antibodies generated by immunizing mice with intact unstimulated HUVEC as prepared in Example 4 were tested for their ability to inhibit the binding of ¹²⁵I-fibrinogen to HUVEC either unstimulated or stimulated with TNF. The binding assays were performed as described in Example 3A with the exceptions described in Example 4 for the screening of hybridoma culture supernatants. HUVEC were exposed to 5 ng/ml TNF for 4 hours at 37°C prior to admixture of the antibodies. After the stimulation, the cells were washed once with the HUVEC culture medium prepared in Example 2A. The monoclonal antibodies, affinity purified 14E11, anti-ICAM-1 BD, and PMI-I (all described in Example 4), were separately admixed at a concentration of 20 *µ*g/ml to either stimulated or unstimulated HUVEC cultures and maintained for 30 minutes at 37°C. After the antibody-reacted cells were washed once with culture medium, 50 *µ*g/ml of ¹²⁵I-labelled fibrinogen prepared in Example 3A was admixed to each well and maintained for 30 minutes at 22*µ*C. The cells were subsequently washed and solubilized as described in Example 3A.

The resultant data is shown in Figures 9A and Figures 9B which respectively shown the effects of antibody exposure on binding of ¹²⁵I-labelled fibrinogen to unstimulated and TNF-stimulated HUVEC. The data is expressed in a bar graph as the specific binding of ¹²⁵I-labelled fibrinogen in cpm/well (X 10-3) on the Y-axis against the specific treatments on the X-axis. As shown in Figure 9A, 14E11 partially inhibited the binding of fibrinogen while the BD anti-ICAM-1 antibody was slightly more effective. However, in the TNF-stimulated HUVEC cultures shown in Figure 9B, 14E11 completely inhibited the binding while the BD antibody was only partially effective. The control antibody, PMI-I, was not inhibitory as compared to the amount of binding in the absence of any antibody. The results, therefore, support the finding that 14E11 specifically recognizes a fibrinogen receptor (or binding site) on the surface of HUVEC the occupation of which completely inhibits the binding of fibrinogen to TNF-stimulated HUVEC. This in vitro system mimics that found in vivo and thus the antibodies of this invention would be useful as therapeutics.

### 6. preparation of Anti-Fibrinogen Monoclonal Antibodies that Block the Binding of Fibrinogen to the RGD-Independent Fibrinogen Receptor on Endothelial Cells

### A. Preparation of Immunoaen

Fibrinogen and the fragments D and E prepared therefrom as described in Example 1, are prepared for use as immunogens in order to raise monoclonal antibodies against the portion of fibrinogen that mediates the binding of fibrinogen to the RGD-independent fibrinogen binding site on endothelial cells. For the immunizations, 50 *µ*g of separately prepared fibrinogen immunogens are admixed in complete Freund's adjuvant (CFA)

### B. Preparation of Monoclonal Antibodies to the Fibrinoqen Site that Mediates the Binding of Fibrinogen on HUVEC

The HUVEC, prepared as immunogens according to Example 4A, are injected intraperitoneally (i.p.) into separate Balb/c ByJ mice followed by a second and third immunization using the same fibrinogen immunogens, each about three weeks apart, in incomplete Freund's adjuvant (IFA). The mice receive a boost of 50 *µ* g of prepared fibrinogen immunogens intravenously (i.v.) in normal saline four days prior to fusion and a second similar perfusion boost one day later.

The animals so treated are sacrificed and the spleen of each mouse is harvested. A spleen cell suspension is then prepared and subjected to the fusion protocol as described in Example 4. Growing clones are then screened for the expression of hybridomas having the selected specificity as described below.

### C. Immunoscreening of Monoclonal Antibodies by Binding Assays

The culture supernatants from HAT resistant cultures prepared above are subsequently assayed for the presence of fibrinogen antibodies that function to inhibit the binding of fibrinogen to the RGD-independent fibrinogen receptor on endothelial cells. The supernatants are screened by both the inhibition of ¹²⁵I-labelled fibrinogen binding assay and the inhibition of ⁵¹Cr-labelled and fibrinogen-bound THP-1 cell attachment assay as described in Example 4C, which are based on the assays described in Example 3A and 3B. The inhibition of binding assay is further described in Example 5.

Hybridoma culture supernatants that produce an antibody that binds to the site on fibrinogen that mediates the binding of fibrinogen to the RGD-independent fibrinogen receptor on endothelial cells (also referred to as ICAM-1 based on the analysis in Examples 2 - 5 are then selected for subsequent purification and characterization. Hybridoma cultures producing antibodies against fibrinogen are identified. The monoclonal antibodies, specific for a region on fibrinogen that binds to the RGD-independent fibrinogen receptor on endothelial cells (ICAM-1) shown to immunoreact with fibrinogen in addition to the analogs thereof, and to not immunoreact with the site on fibrinogen that mediates the binding to MAC-1, namely the site defined by the D₃₀-derived peptides described in Example 3.

### D. Purification of the Selected Monoclonal Antibodies

The selected hybridomas secreting anti-fibrinogen antibodies as described in Example 6C are injected into 10-week old Balb/c mice as described in Example 4D produce ascites fluid.

The ascites-produced monoclonal antibodies are further purified by fast protein liquid chromatography (FPLC) using a Pharmacia Mono Q HR5/5 anion exchange column (Pharmacia) using a 0-0.5 M NaCl gradient in 10 mM Tris-HCl at pH 8.0 following directions supplied with the column. The FPLC-treated monoclonal antibodies are then concentrated using an Amicon stirred ultrafiltration cell (Danvers, MA; PM 30 membrane) to a concentration of 1 mg/ml, dialyzed into PBS and stored at -70°C to form purified monoclonal antibody.

The monoclonal antibodies is further affinity purified using a affinity purification kit, Affi-Prep, according to the manufacturer's instructions (Bio-Rad, Richmond, CA). The IgM monoclonal antibodies are further purified by hydroxylapatite gel filtration over a Bio-Gel HPHT hydroxylapatite column according to manufacturer's instructions (Bio-Rad). These purified antibodies are used in subsequent binding assays, Western immunoblots and immunoprecipitations for use in this invention.

The foregoing specification, including the specific embodiments and examples, is intended to be illustrative of the present invention and is not to be taken as limiting. Numerous other variations and modifications can be effected without departing from the true scope of the present invention, as defined in the attached claims.

## Claims

1. A composition comprising a therapeutically effective amount of a substantially pure and pharmaceutically acceptable fibrinogen homolog capable of binding to ICAM-1 and inhibiting Fg binding to endothelial cells, provided that said homolog is not D₃₀ or a variant thereof.

2. The composition of claim 1 wherein said therapeutically effective amount is at least about 0.1 percent by weight fibrinogen homolog per weight of total composition.

3. The composition of claim 1 wherein said fibrinogen homolog is dispersed in a pharmaceutically acceptable excipient.

4. The composition of claim 1 wherein said fibrinogen homolog is dispersed in a sterile solution.

5. The composition of claim 1 wherein said fibrinogen homolog is selected from the group consisting of a proteolytic fragment of fibrinogen and a monoclonal antibody that immunoreacts with ICAM-1, but does not immunoreact with the vitronectin receptor, wherein said monoclonal antibody preferentially inhibits fibrinogen binding to stimulated endothelial cells.

6. The composition of claim 5 wherein said monoclonal antibody has the immunospecificity of a monoclonal antibody produced by a hybridoma selected from the group consisting of 14E11, 16G8, 2E12, and 2B12.

7. A composition comprising a therapeutically effective amount of a substantially pure and pharmaceutically acceptable ICAM-1 homolog capable of binding to fibrinogen and inhibiting fibrinogen binding to endothelial cells.

8. A monoclonal antibody that immunoreacts with ICAM-1, but does not immunoreact with the vitronectin receptor, wherein said monoclonal antibody preferentially inhibits fibrinogen binding to stimulated endothelial cells.

9. The monoclonal antibody of claim 8 wherein said antibody has the immunospecificity of a monoclonal antibody produced by a hybridoma selected from the group consisting of 14E11, 16G8, 2E12, and 2B12.

10. A monoclonal antibody that immunoreacts with fibrinogen and that preferentially inhibits fibrinogen binding to stimulated endothelial cells.

11. The use of a fibrinogen (Fg) homolog or an ICAM-1 homolog in the manufacture of a medicament for inhibiting Fg binding to endothelial cells in a patient, said Fg homolog capable of binding to ICAM-1 and inhibiting Fg binding to endothelial cells, and said ICAM-1 homolog capable of binding to fibrinogen and inhibiting fibrinogen binding to endothelial cells.

12. The use of claim 11 wherein said Fg holomog is selected from the group consisting of a proteolytic fragment of fibrinogen, D₃₀, and a monoclonal antibody that immunoreacts with ICAM-1, but does not immunoreact with the vitronectin receptor, wherein said monoclonal antibody preferentially inhibits fibrinogen binding to stimulated endothelial cells.

13. The use of claim 12 wherein said monoclonal antibody has the immunospecificity of a monoclonal antibody produced by a hybridoma selected from the group consisting of 14E11, 16G8, 2E12, and 2B12.

14. The use of claim 11 wherein said ICAM-1 homolog is selected from the group consisting of ICAM-1 and a monoclonal antibody comprising antibody molecules that immunoreact with fibrinogen and that preferentially inhibit fibrinogen binding to stimulated endothelial cells.

15. The use of claim 11 wherein said medicament comprises said analog in an amount sufficient to produce an intravascular concentration of homolog in the blood of said patient in the range of about 0.1 to 100 ug/ml.

16. The method of claim 11 wherein said medicament is for administration in an amount to provide from about 0.1 to about 20 milligrams of homolog per kilogram of bodyweight of said patient per day.

17. The use of a Fg homolog or an ICAM-1 homolog in the manufacture of a medicament for inhibiting fibrinogen/endothelial cell-mediated inflammation in a patient, said Fg homolog capable of binding to ICAM-1 and inhibiting Fg binding to endothelial cells, and said ICAM-1 homolog capable of binding to fibrinogen and inhibiting fibrinogen binding to endothelial cells.

18. The use of claim 17 wherein said Fg homolog is selected from the group consisting of a proteolytic fragment of fibrinogen, D₃₀, and a monoclonal antibody that immunoreacts with ICAM-1, but does not immunoreact with the vitronectin receptor, wherein said monoclonal antibody preferentially inhibits fibrinogen binding to stimulated endothelial cells.

19. The use of claim 17 wherein said ICAM-1 homolog is selected from the group consisting of ICAM-1 and a monoclonal antibody comprising antibody molecules that immunoreact with fibrinogen and that preferentially inhibit fibrinogen binding to stimulated endothelial cells.

20. The use of claim 17 wherein said medicament comprises said homolog in an amount sufficient to produce an intravascular concentration of said homolog in the blood of said patient in the range of about 0.1 to about 100 ug/ml.

21. A method of detecting the amount of a fibrinogen (Fg) homolog in a liquid sample comprising:
(a) admixing a sample of stimulated endothelial cells with a predetermined amount of a liquid sample containing a Fg homolog and a predetermined amount of labelled Fg homolog to form a competition reaction admixture;
(b) maintaining said reaction admixture for a predetermined time period sufficient for any Fg homolog present in said composition to bind to said endothelial cells and form an endothelial cell:Fg homolog complex and to allow said labelled Fg homolog to bind to said endothelial cells to form a labelled endothelial cell:Fg homolog complex; and
(c) assaying for the amount of labelled endothelial cell:Fg homolog complex formed in step (b) thereby detecting the amount of a Fg homolog in said composition.

22. The method of claim 21 wherein said labelled fibrinogen homolog is selected from the group consisting of fibrinogen, a proteolytic fragment of fibrinogen, D₃₀, and a monoclonal antibody that immunoreacts with ICAM-1, but does not immunoreact with the vitronectin receptor, wherein said monoclonal antibody preferentially inhibits fibrinogen binding to stimulated endothelial cells.

23. A method of screening for compositions effective at inhibiting fibrinogen binding to ICAM-1 comprising the steps of:
a) admixing in an inhibition reaction admixture preselected amounts of a putative inhibitor composition, a fibrinogen homolog, and an ICAM-1 homolog, wherein said Fg homolog is capable of binding to ICAM-1 and inhibiting Fg binding to endothelial cells, and said ICAM-1 homolog is capable of binding to fibrinogen and inhibiting fibrinogen binding to endothelial cells;
b) maintaining said admixture under conditions sufficient for said ICAM-1 homolog to bind to said Fg homolog and form an ICAM-1 homolog:Fg homolog complex; and
c) measuring the amount of ICAM-1 homolog:Fg homolog complex formed in step (b), and thereby the effectiveness of said inhibitor composition.

24. The method of claim 23 wherein said Fg homolog is selected from the group consisting of fibrinogen, a proteolytic fragment of fibrinogen, D₃₀, and a monoclonal antibody that immunoreacts with ICAM-1, but does not immunoreact with the vitronectin receptor, wherein said monoclonal antibody preferentially inhibits fibrinogen binding to stimulated endothelial cells.

25. The method of claim 23 wherein said ICAM-1 homolog is selected from the group consisting of ICAM-1 and a monoclonal antibody comprising antibody molecules that immunoreact with fibrinogen and that preferentially inhibit fibrinogen binding to stimulated endothelial cells.

26. A method for preparing substantially pure ICAM-1 comprising the steps of:
(a) providing an aqueous detergent composition containing at least ICAM-1;
(b) contacting said ICAM-1-containing composition with a fibrinogen-immobilized matrix comprising fibrinogen affixed to a solid support, said contacting being under conditions sufficient for said ICAM-1 to bind to said fibrinogen and form a solid phase ICAM-1:fibrinogen complex;
(c) washing said solid support and said complex with an aqueous wash buffer comprising Mg⁺⁺, Mn⁺⁺ and an RGD-containing polypeptide under conditions sufficient to elute any proteins bound to fibrinogen in an RGD-dependent manner, said wash buffer being substantially free for Ca⁺⁺; and
(d) eluting said ICAM-1 from said solid support using an aqueous buffer comprising Mg⁺⁺, Mn⁺⁺ and EDTA, to form said substantially pure ICAM-1.

## Patentansprüche

1. Eine Zusammensetzung, enthaltend eine therapeutisch wirksame Menge eines im Wesentlichen reinen und pharmazeutisch annehmbaren Fibrinogen-Homologs, das zur Bindung an ICAM-1 und zur Hemmung der Fg-Bindung an Endothelzellen befähigt ist, mit der Maßgabe, dass dieses Homolog nicht D₃₀ oder eine Variante davon ist.

2. Die Zusammensetzung nach Anspruch 1, worin die therapeutisch wirksame Menge wenigstens etwa 0,1 Gew.-% Fibrinogen-Homolog, bezogen auf das Gewicht der Gesamtzusammensetzung, beträgt.

3. Die Zusammensetzung nach Anspruch 1, worin das Fibrinogen-Homologe in einem pharmazeutisch annehmbaren Träger dispergiert ist.

4. Die Zusammensetzung nach Anspruch 1, worin das Fibrinogen-Homolog in einer sterilen Lösung dispergiert ist.

5. Die Zusammensetzung nach Anspruch 1, worin das Fibrinogen-Homolog ausgewählt ist aus der Gruppe bestehend aus einem proteolytischen Fragment von Fibrinogen und einem monoklonalen Antikörper, der mit ICAM-1 immunreagiert, aber nicht mit dem Vitronectin-Rezeptor immunreagiert, worin der monoklonale Antikörper vorzugsweise eine Fibrinogen-Bindung an stimulierte Endothelzellen hemmt.

6. Die Zusammensetzung nach Anspruch 5, worin der monoklonale Antikörper die Immunspezifizität eines durch ein Hybridom hergestellten Antikörpers, ausgewählt aus der Gruppe bestehend aus 14E11, 16G8, 2E12 und 2B12, hat.

7. Eine Zusammensetzung, enthaltend eine therapeutisch wirksame Menge eines im Wesentlichen reinen und pharmazeutisch annehmbaren ICAM-1-Homologs, das zur Bindung an Fibrinogen und zur Hemmung einer Bindung von Fibrinogen an Endothelzellen befähigt ist.

8. Ein monoklonaler Antikörper, der mit ICAM-1 immunreagiert, aber nicht mit dem Vitronectin-Rezeptor immunreagiert, worin der monoklonale Antikörper vorzugsweise eine Bindung von Fibrinogen an stimulierte Endothelzellen hemmt.

9. Der monoklonale Antikörper nach Anspruch 8, worin der Antikörper die Immunspezifizität eines monoklonalen Antikörpers, hergestellt durch ein Hybridom, ausgewählt aus der Gruppe bestehend aus 14E11, 16G8, 2E12 und 2B12, hat.

10. Ein monoklonaler Antikörper, der mit Fibrinogen immunreagiert und der vorzugsweise eine Bindung von Fibrinogen an stimulierte Endothelzellen hemmt.

11. Die Verwendung eines Fibrinogen-Homologs (Fg-Homologs) oder eines ICAM-1-Homologs zur Herstellung eines Medikaments zur Hemmung einer Fg-Bindung an Endothelzellen in einem Patienten, wobei das Fg-Homolog zur Bindung an ICAM-1 und zur Hemmung einer Fg-Bindung an Endothelzellen befähigt ist, und das ICAM-1-Homolog zur Bindung an Fibrinogen und zur Hemmung einer Bindung von Fibrinogen an Endothelzellen befähigt ist.

12. Die Verwendung nach Anspruch 11, worin das Fg-Homolog ausgewählt ist aus der Gruppe bestehend aus einem proteolytischen Fragment von Fibrinogen, D₃₀ und einem monoklonalen Antikörper, der mit ICAM-1 immunreagiert, aber nicht mit dem Vitronectin-Rezeptor immunreagiert, worin der monoklonale Antikörper vorzugsweise eine Bindung von Fibrinogen an stimulierte Endothelzellen hemmt.

13. Die Verwendung nach Anspruch 12, worin der monoklonale Antikörper die Immunspezifizität eines monoklonalen Antikörpers, hergestellt durch ein Hybridom, ausgewählt aus der Gruppe bestehend aus 14E11, 16G8, 2E12 und 2B12, hat.

14. Die Verwendung nach Anspruch 11, worin das ICAM-1-Homolog ausgewählt ist aus der Gruppe bestehend aus ICAM-1 und einem monoklonalen Antikörper, der Antikörpermoleküle umfasst, die mit Fibrinogen immunreagieren und die vorzugsweise eine Bindung von Fibrinogen an stimulierte Endothelzellen hemmen.

15. Die Verwendung nach Anspruch 11, worin das Medikament das Analog in einer Menge enthält, die ausreichend ist, um eine intravaskuläre Konzentration des Homologs in dem Blut des Patienten in dem Bereich von etwa 0,1 bis 100 µg/ml zu ergeben.

16. Das Verfahren nach Anspruch 11, worin das Medikament zur Verabreichung in einer Menge vorhanden ist, um etwa 0,1 bis etwa 20 Milligramm Homolog pro Kilogramm Körpergewicht des Patienten pro Tag zu ergeben.

17. Die Verwendung eines Fg-Homologs oder eines ICAM-1-Homologs zur Herstellung eines Medikamentes zur Hemmung der durch Fibrinogen/Endothelzellen vermittelten Entzündung in einem Patienten, wobei das Fg-Homolog zur Bindung an ICAM-1 und zur Hemmung einer Fg-Bindung an Endothelzellen befähigt ist, und das ICAM-1-Homolog zur Bindung an Fibrinogen und zur Hemmung einer Bindung von Fibrinogen an Endothelzellen befähigt ist.

18. Die Verwendung nach Anspruch 17, worin das Fg-Homolog ausgewählt ist aus der Gruppe bestehend aus einem proteolytischen Fragment von Fibrinogen, D₃₀ und einem monoklonalen Antikörper, der mit ICAM-1 immunreagiert, aber nicht mit dem Vitronectin-Rezeptor immunreagiert, worin der monoklonale Antikörper vorzugsweise eine Bindung von Fibrinogen an stimulierte Endothelzellen hemmt.

19. Die Verwendung nach Anspruch 17, worin das ICAM-1-Homolog ausgewählt ist aus der Gruppe bestehend aus ICAM-1 und einem monoklonalen Antikörper, der Antikörpermoleküle umfasst, die mit Fibrinogen immunreagieren und die vorzugsweise eine Bindung von Fibrinogen an stimulierte Endothelzellen hemmen.

20. Die Verwendung nach Anspruch 17, worin das Medikament das Homolog in einer Menge enthält, die ausreichend ist, um eine intravaskuläre Konzentration des Homologs in dem Blut des Patienten in dem Bereich von etwa 0,1 bis etwa 100 µg/ml zu ergeben.

21. Ein Verfahren zum Nachweisen der Menge eines Fibrinogen-Homologs (Fg-Homologs) in einer flüssigen Probe, umfassend:
(a) Das Vermischen einer Probe von stimulierten Endothelzellen mit einer vorbestimmten Menge einer flüssigen Probe, die ein Fg-Homolog und eine vorbestimmte Menge eines markierten Fg-Homologs enthält, um eine Konkurrenzreaktionsmischung zu bilden;
(b) Das Aufrechterhalten dieser Reaktionsmischung für einen vorbestimmten Zeitraum, der ausreichend ist, dass das gesamte, in der Zusammensetzung vorhandene Fg-Homolog an die Endothelzellen bindet und einen Endothelzelle : Fg-Homolog-Komplex bildet, und dass das markierte Fg-Homolog an die Endothelzellen binden gelassen wird, um einen markierten Endothelzelle : Fg-Homolog-Komplex zu bilden; und
(c) Das Bestimmen der Menge des in Schritt (b) gebildeten markierten Endothelzelle : Fg-Homolog-Komplexes, wodurch die Menge eines Fg-Homologs in der Zusammensetzung nachgewiesen wird.

22. Das Verfahren nach Anspruch 21, worin das markierte Fibrinogen-Homolog ausgewählt ist aus der Gruppe bestehend aus einem proteolytischen Fragment von Fibrinogen, D₃₀ und einem monoklonalen Antikörper, der mit ICAM-1 immunreagiert, aber nicht mit dem Vitronectin-Rezeptor immunreagiert, worin der monoklonale Antikörper vorzugsweise eine Bindung von Fibrinogen an stimulierte Endothelzellen hemmt.

23. Ein Verfahren zum Durchmustern auf Zusammensetzungen, die zur Hemmung einer Bindung von Fibrinogen an ICAM-1 wirksam sind, umfassend die Schritte:
a) Das Vermischen ausgewählter Mengen einer mutmaßlichen Hemmerzusammensetzung, eines Fibrinogen-Homologs und eines ICAM-1-Homologs in einer Hemmungsreaktionsmischung, worin das Fg-Homolog zur Bindung an ICAM-1 und zur Hemmung einer Bindung von Fg an Endothelzellen befähigt ist, und das ICAM-1-Homolog zur Bindung an Fibrinogen und zur Hemmung einer Bindung von Fibrinogen an Endothelzellen befähigt ist;
b) Das Aufrechterhalten dieser Mischung unter Bedingungen, die ausreichend sind, dass das ICAM-1-Homolog an das Fg-Homolog bindet und einen ICAM-1-Homolog : Fg-Homolog-Komplex bildet; und
c) Das Messen der in Schritt (b) gebildeten Menge des ICAM-1-Homolog : Fg-Homolog-Komplexes und dadurch der Wirksamkeit der Hemmerzusammensetzung.

24. Das Verfahren nach Anspruch 23, worin das Fg-Homolog ausgewählt ist aus der Gruppe bestehend aus Fibrinogen, einem proteolytischen Fragment von Fibrinogen, D₃₀ und einem monoklonalen Antikörper, der mit ICAM-1 immunreagiert, aber nicht mit dem Vitronectin-Rezeptor immunreagiert, worin der monoklonale Antikörper vorzugsweise eine Bindung von Fibrinogen an stimulierte Endothelzellen hemmt.

25. Das Verfahren nach Anspruch 23, worin das ICAM-1-Homolog ausgewählt ist aus der Gruppe bestehend aus ICAM-1 und einem monoklonalen Antikörper, welcher Antikörpermoleküle umfasst, die mit Fibrinogen immunreagieren und die vorzugsweise eine Bindung von Fibrinogen an stimulierte Endothelzellen hemmen.

26. Ein Verfahren zur Herstellung von im Wesentlichen reinem ICAM-1, umfassend die Schritte:
(a) Das Bereitstellen einer wenigstens ICAM-1 enthaltenden wässrigen Detergenszusammensetzung;
(b) Das Inberührungbringen der ICAM-1 enthaltenden Zusammensetzung mit einer Fibrinogen immobilisierten Matrix, enthaltend an einen festen Träger gebundenes Fibrinogen, wobei das Inberührungbringen unter Bedingungen erfolgt, die ausreichend sind, dass das ICAM-1 an das Fibrinogen bindet und einen Festphasen-ICAM-1 : Fibrinogen-Komplex bildet;
(c) Das Waschen des festen Trägers und des Komplexes mit einem wässrigen Waschpuffer, enthaltend Mg⁺⁺, Mn⁺⁺ und ein RGD enthaltendes Polypeptid, unter Bedingungen, die ausreichend sind, um sämtliche in einer RGD-abhängigen Weise an Fibrinogen gebundene Proteine zu eluieren, wobei der Waschpuffer im Wesentlichen frei ist von Ca⁺⁺; und
(d) Das Eluieren des ICAM-1 von dem festen Träger unter Verwendung eines wässrigen, Mg⁺⁺, Mn⁺⁺ und EDTA enthaltenden Puffers, um im Wesentlichen reines ICAM-1 zu bilden.

## Revendications

1. Composition comprenant une quantité thérapeutiquement efficace d'un homologue de fibrinogène substantiellement pur et pharmaceutiquement acceptable capable de se lier à ICAM-1 et d'inhiber la liaison de Fg aux cellules endothéliales à condition que ledit homologue ne soit pas D₃₀ ou un variant.

2. Composition de la revendication 1 où ladite quantité thérapeutiquement efficace est d'au moins environ 0,1 pour cent en poids d'homologue de fibrinogène par poids de la composition totale.

3. Composition de la revendication 1 où ledit homologue de fibrinogène est dispersé dans un excipient pharmaceutiquement acceptable.

4. Composition de la revendication 1, où ledit homologue de fibrinogène est dispersé dans une solution stérile.

5. Composition de la revendication 1 où ledit homologue de fibrinogène est sélectionné dans le groupe consistant en un fragment protéolytique de fibrinogène et un anticorps monoclonal qui immunoréagit avec ICAM-1, mais n'immunoréagit pas avec le récepteur de vitronectine, où ledit anticorps monoclonal inhibe préférentiellement la liaison du fibrinogène aux cellules endothéliales stimulées.

6. Composition de la revendication 5, où ledit anticorps monoclonal a l'immunospécificité d'un anticorps monoclonal produit par un hybridome sélectionné dans le groupe consistant en 14E11, 16G8, 2E12, et 2B12.

7. Composition comprenant une quantité thérapeutiquement efficace d'un homologue de ICAM-1 substantiellement pur et pharmaceutiquement acceptable capable de se lier au fibrinogène et inhibant la liaison du fibrinogène aux cellules endothéliales.

8. Anticorps monoclonal qui immunoréagit avec ICAM-1 mais n'immunoréagit pas avec le récepteur de vitronectine, où ledit anticorps monoclonal inhibe préférentiellement la liaison du fibrinogène aux cellules endothéliales stimulées.

9. Anticorps monoclonal de la revendication 8 où ledit anticorps a l'immunospécificité d'un anticorps monoclonal produit par un hybridome sélectionné dans le groupe consistant en 14E11, 16G8, 2E12 et 2B12.

10. Anticorps monoclonal qui immunoréagit avec le fibrinogène et qui inhibe préférentiellement la liaison du fibrinogène aux cellules endothéliales stimulées.

11. Utilisation d'un homologue de fibrinogène (Fg) ou d'un homologue de ICAM-1 dans la fabrication d'un médicament pour inhiber la liaison de Fg à des cellules endothéliales chez un patient, ledit homologue de Fg capable de se lier à ICAM-1 et d'inhiber la liaison de Fg aux cellules endothéliales et ledit homologue de ICAM-1 capable de se lier au fibrinogène et inhibant la liaison du fibrinogène aux cellules endothéliales.

12. Utilisation de la revendication 11, où ledit homologue de Fg est sélectionné dans le groupe consistant en un fragment protéolytique du fibrinogène, D₃₀, et un anticorps monoclonal qui immunoréagit avec ICAM-1, mais n'immunoréagit pas avec le récepteur de vitronectine, où ledit anticorps monoclonal inhibe préférentiellement la liaison du fibrinogène aux cellules endothéliales stimulées.

13. Utilisation de la revendication 12 où ledit anticorps monoclonal a l'immunospécificité d'un anticorps monoclonal produit par un hybridome sélectionné dans le groupe consistant en 14E11, 16G8, 2E12, et 2B12.

14. Utilisation de la revendication 11 où ledit homologue de ICAM-1 est sélectionné dans le groupe consistant en ICAM-1 et un anticorps monoclonal comprenant des molécules d'anticorps qui immunoréagissent avec le fibrinogène et qui inhibent préférentiellement la liaison du fibrinogène aux cellules endothéliales stimulées.

15. Utilisation de la revendication 11 où ledit médicament comprend ledit analogue en une quantité suffisante pour produire une concentration intravasculaire de l'homologue dans le sang dudit patient dans la gamme d'environ 0,1 à 100 µg/ml.

16. Méthode de la revendication 11 où ledit médicament est pour administration en une quantité pour produire environ 0,1 à environ 20 milligrammes de l'homologue par kilogramme de poids corporel dudit patient par jour.

17. Utilisation d'un homologue de Fg ou d'un homologue de ICAM-1 dans la fabrication d'un médicament pour inhiber l'inflammation due au fibrinogène/cellules endothéliales chez un patient, ledit homologue de Fg étant capable de se lier à ICAM-1 et inhibant la liaison de Fg aux cellules endothéliales et ledit homologue de ICAM-1 étant capable de se lier au fibrinogène et inhibant la liaison du fibrinogène aux cellules endothéliales.

18. Utilisation de la revendication 17 où ledit homologue de Fg est sélectionné dans le groupe consistant en un fragment protéolytique de fibrinogène, D₃₀, et un anticorps monoclonal qui immunoréagit avec ICAM-1, mais n'immunoréagit pas avec le récepteur de vitronectine, où ledit anticorps monoclonal inhibe préférentiellement la liaison du fibrinogène aux cellules endothélailes stimulées.

19. Utilisation de la revendication 17, où ledit homologue de ICAM-1 est sélectionné dans le groupe consistant en ICAM-1 et un anticorps monoclonal comprenant des molécules d'anticorps qui immunoréagissent avec le fibrinogène et qui inhibent préférentiellement la liaison du fibrinogène à des cellules endothéliales stimulées.

20. Utilisation de la revendication 17 où ledit médicament comprend ledit homologue en une quantité suffisante pour produire une concentration intravasculaire dudit homologue dans le sang dudit patient dans la gamme d'environ 0,1 à environ 100 µg/ml.

21. Méthode de détection de la quantité d'un homologue de fibrinogène (Fg) dans un échantillon liquide comprenant:
(a) le mélange d'un échantillon de cellules endothéliales stimulées avec une quantité prédéterminée d'un échantillon liquide contenant un homologue de Fg et une quantité prédéterminée d'un homologue de Fg marqué pour former un un mélange réactionnel de compétition;
(b) le maintien dudit mélange réactionnel pendant une période prédéterminée de temps suffisante pour que tout homologue de Fg présent dans ladite composition se lie auxdites cellules endothéliales et forme un complexe cellules endothéliales: homologue de Fg et pour permettre audit homologue de Fg marqué de se lier auxdites cellules endothéliales pour former un complexe cellules endothéliales: homologue de Fg marqué; et
(c) l'analyse de la quantité du complexe cellules endothéliales: homologue de Fg marqué formé à l'étape (b) pour ainsi détecter la quantité d'un homologue de Fg dans ladite composition.

22. Méthode de la revendication 21, où ledit homologue de fibrinogène marqué est sélectionné dans le groupe consistant en fibrinogène, un fragment protéolytique du fibrinogène, D₃₀, et un anticorps monoclonal qui immunoréagit avec ICAM-1, mais n'immunoréagit pas avec le récepteur de vitronectine, où ledit anticorps monoclonal inhibe préférentiellement la liaison du fibrinogène aux cellules endothéliales stimulées.

23. Méthode de criblage de compositions efficaces pour inhiber la liaison du fibrinogène à ICAM-1 comprenant les étapes de:
a) mélanger, dans un mélange réactionnel d'inhibition, des quantités présélectionnées d'une composition d'un inhibiteur putatif, d'un homologue de fibrinogène, et d'un homologue de ICAM-1, où ledit homologue de F9 est capable de se lier à ICAM-1 et d'inhiber la liaison de Fg à des cellules endothéliales et ledit homologue de ICAM-1 est capable de se lier au fibrinogène et d'inhiber la liaison du fibrinogène aux cellules endothéliales; et
b) maintenir ledit mélange dans des conditions suffisantes pour que ledit homologue de ICAM-1 se lie audit homologue de Fg et forme un complexe homologue de ICAM-1: homologue de Fg; et
c) mesurer la quantité du complexe homologue de ICAM-1: homologue de Fg formé à l'étape (b), et ainsi l'efficacité de ladite composition de l'inhibiteur.

24. Méthode de la revendication 23, où ledit homologue de Fg est sélectionné dans le groupe consistant en fibrinogène, un fragment protéolytique du fibrinogène, D₃₀, et un anticorps monoclonal qui immunoréagit avec ICAM-1, mais n'immunoréagit pas avec le récepteur de vitronectine, où ledit anticorps monoclonal inhibe préférentiellement la liaison du fibrinogène aux cellules endothéliales stimulées.

25. Méthode de la revendication 23, où ledit homologue de ICAM-1 est sélectionné dans le groupe consistant en ICAM-1 et un anticorps monoclonal comprenant des molécules d'anticorps qui immunoréagissent avec le fibrinogène et qui inhibent préférentiellement la liaison du fibrinogène aux cellules endothéliales stimulées.

26. Méthode de préparation de ICAM-1 sensiblement pur, comprenant les étapes de :
(a) prévoir une composition aqueuse d'un détergent contenant au moins ICAM-1;
(b) mettre ladite composition contenant ICAM-1 en contact avec une matrice immobilisée-fibrinogène comprenant du fibrinogène fixé à un support solide, ledit contact étant dans les conditions suffisantes pour que ICAM-1 se lie audit fibrinogène et forme un complexe ICAM-1:fibrinogène en phase solide;
(c) laver ledit support solide et ledit complexe avec un tampon aqueux de lavage comprenant Mg⁺⁺, Mn⁺⁺ et un polypeptide contenant RGD dans des conditions suffisantes pour éluer toutes les protéines liées au fibrinogène d'une manière dépendant de RGD, ledit tampon de lavage étant sensiblement libre de Ca⁺⁺; et
(d) éluer ledit ICAM-1 dudit support solide en utilisant un tampon aqueux comprenant Mg⁺⁺, Mn⁺⁺ et EDTA, pour former ledit ICAM-1 sensiblement pur.
